(19) Europäisches Patentamt
European Patent Office
Office européen des brevets

(11) EP 1 594 472 B1

(12) # EUROPEAN PATENT SPECIFICATION

(45) Date of publication and mention of the grant of the patent:
**05.12.2007 Bulletin 2007/49**

(21) Application number: **04713577.7**

(22) Date of filing: **23.02.2004**

(51) Int Cl.:
***A61K 9/16*** *(2006.01)*

(86) International application number:
**PCT/GB2004/000698**

(87) International publication number:
**WO 2004/073688 (02.09.2004 Gazette 2004/36)**

(54) **DELIVERY OF A NSAID FROM EMBOLIC AGENTS**

ABGABE EINES NSAID AUS EMBOLISCHEN MITTELN

ADMINISTRATION D'UN AINS A PARTIR D'AGENTS EMBOLIQUES

(84) Designated Contracting States:
**AT BE BG CH CY CZ DE DK EE ES FI FR GB GR HU IE IT LI LU MC NL PT RO SE SI SK TR**

(30) Priority: **21.02.2003 EP 03251075**

(43) Date of publication of application:
**16.11.2005 Bulletin 2005/46**

(73) Proprietor: **Biocompatibles UK Limited**
**Farnham**
**Surrey GU9 8QL (GB)**

(72) Inventors:
- **LEWIS, Andrew, Lennard,**
**Biocompatibles UK Limited**
**Farnham,**
**Surrey GU9 8QL (GB)**
- **STRATFORD, Peter, William,**
**Biocompatibles UK Ltd.**
**Farnham,**
**Surrey GU9 8QL (GB)**
- **LEPPARD, Simon, W.,**
**Biocompatibles UK Limited**
**Farnham,**
**Surrey GU9 8QL (GB)**

(74) Representative: **Jones, Helen M.M.**
**Gill Jennings & Every LLP**
**Broadgate House**
**7 Eldon Street**
**London EC2M 7LH (GB)**

(56) References cited:
**WO-A-00/23054**
**WO-A-00/28920**
**WO-A-01/68720**
**WO-A-99/12577**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 594 472 B1

Printed by Jouve, 75001 PARIS (FR)

## Description

The present invention relates to compositions which embolise uterine fibroids and deliver drugs at the site of embolisation. The drugs are non-steroidal anti-inflammatory drugs (NSAID's) and have cyclooxygenase (COX) inhibitory properties which will reduce inflammation caused by embolisation.

Embolisation therapy involves the introduction of an agent into the vasculature in order to bring about the deliberate blockage of a particular vessel. This type of therapy is particularly useful for blocking abnormal connections between arteries and veins (such as arteriovenous malformations, or AVMs), and also for occluding Vessels that feed certain hyper-vascularised tumours, in order to starve the abnormal tissue and bring about its necrosis and shrinkage. One application of embolotherapy that is receiving increasing attention is the treatment of uterine fibroids. Uterine fibroids or leiomyomata are the most common tumour found in women. Fibroids are benign clonal tumours arising from the smooth-muscle cells of the uterus. Approximately 25% of premenopausal women suffer from fibroids, while the overall prevalence of these tumours could be as high as 77%. The incidence of fibroids in African-American women is three times that of Caucasian women. Fibroids may occur at any age, but are most common in women over the age of 40 years. After menopause, fibroids usually regress in size due to the lack of hormonal stimulation, which may result in infarction.

The rationale for utilizing embolisation to treat uterine fibroids can be traced to several known indications for embolotherapy. First, embolisation has been used with success as a palliative treatment in end-stage cancer patients for symptomatic relief. Examples of this include patients with bony metastases arising from renal cell carcinoma and patients with inoperable liver tumours (hepatoma and colon metastases). The reason why this procedure works in this scenario is because depriving a tumour of its blood supply ultimately decreases the size of the tumour, resulting in relief of mass-related symptoms. Second, embolisation has been shown to reduce. the vascularity of tumours prior to surgical excision thereby reducing intraoperative blood loss; this indication has been utilized for renal cell carcinomas and spinal tumours prior to resection. Third, embolisation has been used with success to control tumour-related bleeding in sites throughout the body. Examples of this success include bleeding secondary to renal cell caricinoma, bladder tumours, angiomyolipoma, and hepatic adenomas. Finally, embolisation has been used with success to control abnormal uterine bleeding due to gynecologic malignancies (endometrial, cervical, and ovarian), postpartum bleeding, postsurgical bleeding, bleeding from an ectopic pregnancy and bleeding due to congenital AV malformations. A recent article by Vedantham, et al Appl Radiol, 31(10):9-17, 2002, reviews the indications for uterine artery embolization in the obstetrical and gynecologic patient population.

In the major studies of uterine fibroid embolisation to date, the most frequently used embolic material is particulate polyvinyl alcohol, which has been classified according to its particle size. The gel is delivered in suspension form in an aqueous vehicle, using a micocatheter, delivered to one or both of the uterine arteries.

One drawback to the UFE procedure is the associated pain that may be experienced by the patient. For this reason, conscious sedation and analgesia are critical to the successful outcome of a UFE procedure. Not only does this help to reduce anxiety, but more specifically addresses the severe pelvic pain, cramps, and nausea that is termed postembolisation syndrome. Immediately following the UFE procedure, the patient can use an analgesia pump to self-administer narcotic pain relief. Supplementation with systemic analgesics helps to reduce the amount of narcotic used by combatting pain and cramping. From four of the trials listed by Vedantham *et al,* despite high procedural success, pain is encountered as a major result.

Periprocedural pain control therefore, is of utmost importance since it can represent the major morbidity of the procedure. Pain generally starts early after the embolisation and reaches the highest severity 24 to 48 hours after the embolisation. Most pain protocols use a combination of opioids, such as an oxycodone derivative, and a nonsteroidal anti-inflammatory (NSAID), such as ibuprofen or ketorolac. Successful pain control potentially allows this procedure to be performed on an outpatient basis. Early studies attempting to perform UFE as an outpatient procedure reported that 15% of patients returned to the hospital for pain control. One should not use intraarterial lidocaine in an attempt to reduce pain since it causes a large amount of spasm (Keyoung JA, Levy EB, Roth AR, et al. Intraarterial lidocaine for pain control after uterine artery embolization for leiomyomata. J vasc Intervent Radiol. 2001;12:1065-1069). Postembolization syndrome with severe pain, fever, and an elevation in the white blood count occurs in as many as 34% of patients. (Goodwin SC, McLucas B, Lee M, et al. Uterine artery embolization for the treatment of uterine leiomyomata midterm results. J Vasc Intervent Radiol. 1999; 10 : 1159-1165).

Siskin *et al,* (Siskin GP, Stainken BF, Dowling K, et al. Outpatient uterine artery embolization for symptomatic uterine fibroids: Experience in 49 patients: J Vasc Intervent Radiol. 2000;11:305-311) reported 95.9% successful discharge after 8 hours of post-procedure observation. This however, has been acknowledged as being a very complex pain-management regime comprising of both intravenous IV and oral administrations (Burbak F, et al. J Am Soc Gyn Laparoscopists 7(4), S1-49, 2000). They further elaborated on this atypical observation in (Siskin et al, Techniques in Vascular and Interventional Radiology, 5(1), 35-43, 2002), where they state that the management of pain varies so widely between hospitals, that there is a medical need to keep the patients in hospital for observation during the first

24-48hrs of pain treatment. The ability to discharge patients within the same day is often impossible, and only managed in that the procedure starts early morning, and finishes late in the evening the same day. Observation by the hospital staff is required during the PCA pump delivery of the opiate, and precludes early discharge. Observations of procedures within a Hospital in the UK indicated that the low incidence of UFE treatment was due to the availability of beds for the hospital stay rather than the patients and Interventional Radiologist to carry out the procedure.

Although UFE is considered very safe, any medical procedure has some associated risks. Most patients feel cramping after UFE. The severity of pain varies from patient to patient. Pain is related to the death of the fibroid and to some degree the reduced blood supply (ischemia) to the normal portion of the uterus. The pain is biphasic with the first 2-6 hours of intense pain followed by a second phase of mild to moderate pain that can be short or lasting up to several days. However, not every patient feels pain after embolisation but it is reported to occur in 95% of patients.

The pain is treated actively by starting oral anti-inflammatory drugs 2 hours before the procedure and morphine after the procedure. The morphine is administrated through a PCA (patient controlled analgesia) pump. The patient can push a button to administer the medication in case of pain. When the pain becomes tolerable, and after at least 4-6 hours of bed rest, the patient can leave the hospital. Most of the time the patient spends one night in the hospital.

Non Steroidal Anti-Inflammatory Drugs (NSAIDs) are medications which, as well as having pain-relieving (analgesic) effects, have the effect of reducing inflammation when used over a period of time. A new class of NSAIDs, cyclooxygenase-2 (COX-2) inhibitors, selectively inhibits inflammatory prostaglandins (PGs). These new drugs have a lower complication rate and do not tend to produce ulcers. There are many different types of NSAIDs, including aspirin and other salicylates. Examples include; ibuprofen (Motrin, Advil), naproxen (Naprosyn), diclofenac (Voltaren), ketoprofen (Orudis), indomethacin (Indocin), and newer ones such as celecoxib (Celebrex), the first COX-2 inhibitor on the market, and rofecoxib (Vioxx), which was recently released:

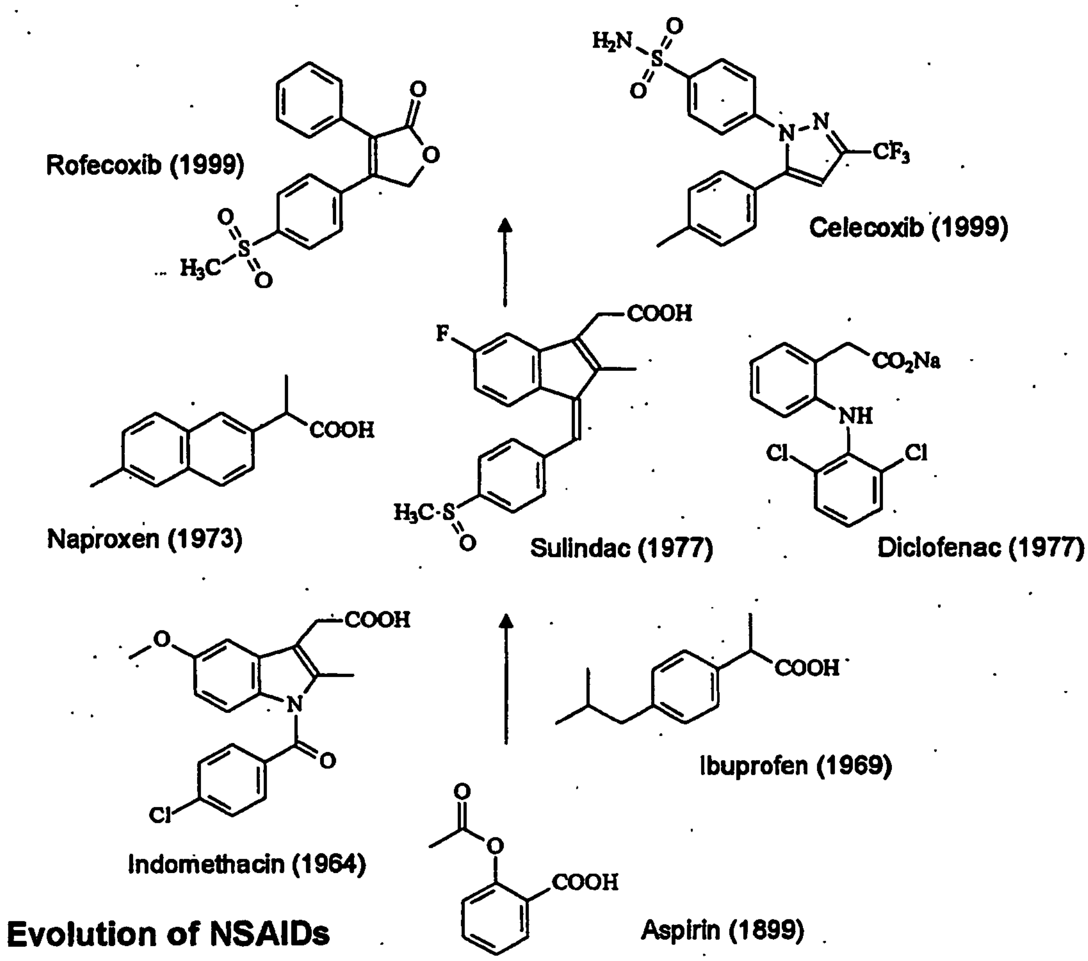

Rofecoxib (1999)

Celecoxib (1999)

Naproxen (1973)

Sulindac (1977)

Diclofenac (1977)

Indomethacin (1964)

Ibuprofen (1969)

Aspirin (1899)

Evolution of NSAIDs

The primary mechanism of action in NSAIDs is by interfering with the cyclooxygenase pathway (enzymes that make prostaglandins) and a resultant decrease in prostaglandin synthesis.

In the female reproductive tract NSAIDs are reported not only to inhibit endometrial prostaglandins, but also improve platelet aggregation and degranulation and increase uterine vasoconstriction in women with menorrhagia (van Eijkeren JJ, 1992). Prostaglandins are active mediators of the inflammatory cascade, which also serve to sensitize peripheral nociceptors (nerve endings). Recent research (Tannenbaum H 1996, Vane JR 1996, Emery P 1996) has shown that there are two types of cyclooxygenase, denoted COX-1 and COX-2. Each type of cyclooxygenase lends itself to producing different types of prostaglandins.

There are two types of prostaglandins.

The first type comprises maintenance Prostaglandins. These are made regularly by the body, are produced by COX-1 enzyme and play a role in maintaining normal function in several organ systems. Examples of maintenance effect in some organs are the protective lining of the stomach, normal platelet function and kidney blood flow.

The second class of prostaglandins are "inflammatory". They are produced by the body in response to an inflammatory stimulus and are produced by COX-2 enzyme. They play a role in causing inflammation and pain.

As mentioned above, there are two types of cyclooygenase enzyme. COX-1 is stimulated continuously by normal body physiology. The COX-1 enzyme is constitutive, meaning that its concentration in the body remains stable. It is present in most tissues and converts arachidonic acid into prostaglandins. The location of the COX-1 enzyme dictates the function of the prostaglandins it releases (Vane JR 1996). For example, COX-1 in the stomach wall produces Prostaglandins that stimulate mucous production. COX-1 performs a housekeeping function to synthesize PGs which regulate normal cell activity

COX-2, in contrast to COX-1, is induced in most parts of the body. It is not normally present in cells but its expression can be increased dramatically by the action of macrophages the scavenger cells of the immune system (Tannenbaum H, 1996). COX-2's most important role is in inflammation. COX-2 is involved in producing Prostaglandins for an inflammatory response. Cyclooxygenase-2 (COX-2), known to be elevated in several human cancers, regulates angiogenesis by inducing production of angiogenic factors (Fujiwaki R, 2002). COX-2 is constitutive in the kidney, ovary, uterus and brain. There is believed to be a link between cancer of the uterus and the COX-2 enzyme. COX-2 and its product Prostaglandins set off a cascade of molecular events, including an abnormal increase in estrogen, that leads to tumor growth. Differential COX localization and PG release in Thy-1 (+) and Thy-1 (-) human female reproductive tract has been reported. COX-2, which is generally considered an inducible form, in the female reproductive tract is constitutively expressed in Thy-1 (-) fibroblast subset, which minimally produces PGE (2). And Thy-1 (+) fibroblasts highly express COX-1, which is responsible for the high-level PGE (2) production, a feature usually attributed to COX-2 (Koumas L, 2002).

Inhibitors of COX have activities against both enzymes but many are selective to one or other of the enzymes.

Inhibitors with high COX-1 selectivity are found to have undesirable side effects on the G1 tract; manifest when delivered orally. The recently launched COX-2 selective inhibitors reduce such side effects when administered orally.

Fibroids are commonly found in women with menorrhagia (an excessive abnormal uterine bleeding) and fibroids of the submucosal type in particular have been associated with menorrhagia. Menorrhagia is characterized by either heavy menstrual bleeding or prolonged menstrual bleeding. Women with fibroids might discharge such heavy volumes of blood during their period that they have to constantly change sanitary protection. At the same time, whereas most women have periods that last 4 to 5 days, a woman with fibroids may bleed for over a week.

Dysmenorrhea is divided into two types: primary (affect young teens) and secondary dysmenorrhea (older women). Both types include the following symptoms: backache, diarrhea, dizziness, headache, nausea, vomiting, and tenseness. Fibroids are one of the conditions which often causes or sparks the development of secondary dysmenorrhea (Gynecological Health Center (B), 1).

Short courses of ibuprofen were successful in reducing pain in pregnant women with painful uterine leiomyomas (Katz VL, 1989). It was reported to suppress menstrual PGF2á release far more than PGE2 compared naproxen, which equally suppressed both types of PGs. Selectivity for PGF2á is suggested to reduce risks of closure of the fetal ductus arteriosus linked to pre-mature labor (Chan WY, 1983, Powell AM, 1984, Chan WY, 1981). Reduction of intra-uterine pressure and pain intensity by using ibuprofen in a dysmenorrhoeic patient has been reported (Milsom 1, 1985, Milsom 1, 1984, Chan WY, 1983). Ibuprofen, mefenamic acid and naproxen significantly reduced bleeding in women with menorrhagia by 30-50% (Anderson ABM, 1976 and Makarainen L, 1986). Clinical relief of the dysmenorrhoeic symptoms by ibuprofen accompanies the reduction of menstrual fluid prostaglandin (Dawood My, 1981). Ibuprofen 1200 mg/day reduced (P less than 0.01) median blood loss in primary menorrhagia, but had no effect on blood loss in women with uterine fibroids and factor VIII deficiency (Makarainen L, Ylikorkala O, 1986). There is a failure rate of~20-25% of using NSAIDs in treatment of dysmenorrhea (Wilson ML, 2001). Their mode of action is thought to be by inhibiting endometrial synthesis of prostaglandins (Sanfilippo Js, 1983).

WO0023054 describes microspheres for use in embolisation comprising poly(vinyl alcohol) cross-linked by reaction with an aldehyde. The microsphere diameters are in the range 10-2000 μm. The suspense of microspheres is used in embolisation for a range of indication including uterine fibroids. The microspheres may comprise an anti-angiogenic agent. Examples of anti-angiogenic agents include diclofenac/hyaluronan, ibuprofen and indomethacin.

WO0168720 discloses embolic agents formed by polymerising poly(vinyl alcohol) macromer either *in situ,* or prior to adminstration as microspheres. Embolic agents are said to be of use to induce atrophy of uterine fibroids. Chemotherapeutic agents or other actives may be incorporated into the embolic agents.

According to the present invention there is provided a new use of polymer and, associated with polymer in a releasable form, a pharmaceutically active agent which is a non-steroidal anti-inflammatory agent as defined in claim 1, in the manufacture of a composition for use in uterine fibroid embolisation, in which the pharmaceutical active is released from the polymer at the site of embolisation.

The active may alternatively be defined as a COX inhibitor.

The invention allows local delivery of appropriate pharmaceutical agents for pain relief and/or antiinflammatory treatment of uterine fibroids via a polymer-based embolic agent. The polymer is a water-insoluble material. Although it may be biodegradable, so that drug may be released substantially by erosion of polymer matrix to release drug from the surface, preferably the polymer is substantially biostable. It is preferred for the polymer to be water-swellable.

Water-swellable polymer useful in the invention preferably has a equilibrium water content, when swollen in water at 37°C, measured by gravimetric analysis, in the range of 40 to 99 wt%, preferably 75 to 95%.

In the invention, the composition which is administered to a patient in need of embolisation therapy, is in the form of a suspension of particles of water-swollen water-insoluble polymer. Preferably the particles are graded into calibrated size ranges for accurate embolisation of vessels. The particles preferably have sizes when equilibrated in water at 37°C, in the range 100 to 1200 μm. The calibrated ranges may comprise particles having diameters with a bandwidth of about 100 to 300 μm. The size ranges may be for instance 100 to 300 μm, 300 to 500 μm, 500 to 700 μm, 700 to 900 μm and 900 to 1200 μm. Preferably the particles are substantially spherical in shape. Such particles are referred to herein as microspheres.

Generally the polymer is covalently crosslinked, although it may be appropriate for the polymer to be ionically crosslinked, at least in part. The polymer may be formed by polymerising ethylenically unsaturated monomers in the presence of di- or higher-functional crosslinking monomers, the ethylenically unsaturated monomers preferably including an ionic (including zwitterionic) monomer. Copolymers of hydroxyethyl methacrylate, acrylic acid and cross-linking monomer, such as ethylene glycol dimethacrylate or methylene bisacrylamide, as used for etafilcon A based contact lenses may be used.

Another type of polymer which may be used to form the water-swellable water-insoluble matrix is polyvinyl alcohol crosslinked using aldehyde type crosslinking agents such as glutaraldehyde. For such products, the polyvinyl alcohol (PVA) may be rendered ionic. For instance the PVA may be rendered ionic by providing pendant ionic groups by reacting a functional ionic group containing compound with the hydroxyl groups. Examples of suitable functional groups for reaction with the hydroxyl groups are acylating agents, such as carboxylic acids or derivatives thereof, or other acidic groups which may form esters.

The invention is of particular value where the polymer matrix is formed of a polyvinyl alcohol macromer, having more than one ethylenically unsaturated pendant group per molecule, by radical polymerisation of the ethylenic groups. Preferably the PVA macromer is copolymerised with ethylenically unsaturated monomers for instance including a nonionic and/or ionic monomer.

The PVA macromer may be formed, for instance, by providing PVA polymer, of a suitable molecular weight such as in the range 1000 to 500,000 D, preferably 10,000 to 100,000 D, with pendant vinylic or acrylic groups. Pendant acrylic groups may be provided, for instance, by reacting acrylic or methacrylic acid with PVA to form ester linkages through some of the hydroxyl groups. Other methods for attaching vinylic groups capable of polymerisation onto polyvinyl alcohol are described in, for instance, US 4,978,713 and, preferably, US 5,508,317 and 5,583,163. Thus the preferred macromer comprises a backbone of polyvinyl alcohol to which is linked, via a cyclic acetal linkage, an (alk)acrylaminoalkyl moiety. Example 1 describes the synthesis of an example of such a macromer known by the approved named nelfilcon B. Preferably the PVA macromers have about 2 to 20 pendant ethylenic groups per molecule, for instance 5 to 10.

Where PVA macromers are copolymerised with ethylenically unsaturated monomers including an ionic monomer, the ionic monomer preferably has the general formula I

$$Y^1BQ$$

in which $Y^1$ is selected from

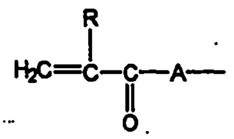 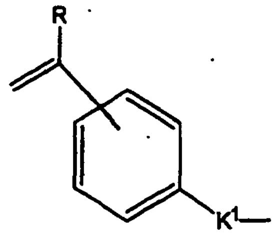

$CH_2=C(R)-CH_2-O-$, $CH_2=C(R)-CH_2$ $OC(O)-$, $CH_2=C(R)OC(O)-$, $CH_2=C(R)-O-$, $CH_2=C(R)CH_2OC(O)N(R^1)-$, $R^2OOCCR=CRC(O)-O-$, $RCH=CHC(O)O-$, $RCH=C(COOR^2)CH_2-C(O)-O-$,

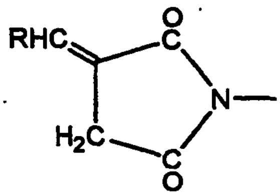 and 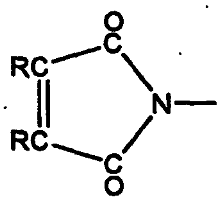

wherein:

R is hydrogen or a $C_1$-$C_4$ alkyl group;
$R^1$ is hydrogen or a $C_1$-$C_4$ alkyl group;
$R^2$ is hydrogen or a $C_{1-4}$ alkyl group or BQ where B and Q are as defined below,
A is-O-or-$NR^1$-;
$K^1$ is a group $-(CH_2)_rOC(O)-$, $-(CH_2)_rC(O)O-$, $-(CH_2)_rOC(O)O-$, $-(CH_2)_rNR^3-$, $-(CH_2)_rNR^3C(O)-$, $-(CH_2)_rC(O)NR^3-$, $-(CH_2)_rNR^3C(O)O-$, $-(CH_2)_rOC(O)NR^3-$, $-(CH_2)_rNR^3C(O)NR^3-$ (in which the groups $R^3$ are the same or different), $-(CH_2)_rO-$, $-(CH_2)_rSO_3-$, or, optionally in combination with $B^1$, a valence bond and r is from 1 to 12 and $R^3$ is hydrogen or a $C_1$-$C_4$ alkyl group;
B is a straight or branched alkanediyl, oxaalkylene, alkanediyloxaalkanediyl, or alkanediyloligo(oxaalkanediyl) chain optionally containing one or more fluorine atoms up to and including perfluorinated chains or, if Q or $Y^1$ contains a terminal carbon atom bonded to B a valence bond; and
Q is an ionic group.

An anionic group Q may be, for instance, a carboxylate, carbonate, sulphonate, sulphate, nitrate, phosphonate or phosphate group. The monomer may be polymerised as the free acid or in salt form. Preferably the $pK_a$ of the conjugate acid is less than 5.

A suitable cationic group Q is preferably a group $N^+R^4{}_3$, $P^+R^5{}_3$ or $S^+R^5{}_2$ in which the groups $R^4$ are the same or different and are each hydrogen, $C_{1-4}$-alkyl or aryl (preferably phenyl) or two of the groups $R^4$ together with the heteroatom to which they are attached from a saturated or unsaturated heterocyclic ring containing from 5 to 7 atoms the groups $R^5$ are each $OR^4$ or $R^4$. Preferably the cationic group is permanently cationic, that is each $R^4$ is other than hydrogen. Preferably a cationic group Q is $N^+R^4{}_3$ in which each $R^4$ is $C_{1-4}$-alkyl, preferably methyl.

A zwitterionic group Q may have an overall charge, for instance by having a divalent centre of anionic charge and monovalent centre of cationic charge or vice-versa or by having two centres of cationic charge and one centre of anionic charge or vice-versa. Preferably, however, the zwitterion has no overall charge and most preferably has a centre of monovalent cationic charge and a centre of monovalent anionic charge.

Examples of zwitterionic groups which may be used as Q in the present invention are disclosed in WO-A-0029481.

Where the ethylenically unsaturated monomer includes zwitterionic monomer, for instance, this may increase the hydrophilicity, lubricity, biocompatibility and/or haemocompatibility of the particles. Suitable zwitterionic monomers are described in our earlier publications WO-A-9207885, WO-A-9416748, WO-A-9416749 and WO-A-9520407. Preferably a zwitterionic monomer is 2-methacryloyloxy-2'-trimethylammonium ethyl phosphate inner salt (MPC).

In the monomer of general formula I preferably $Y^1$ is a group $CH_2=CRCOA-$ in which R is H or methyl, preferably methyl, and in which A is preferably NH. B is preferably an alkanediyl group of 1 to 12, preferably 2 to 6 carbon atoms.

Such monomers are acrylic monomers.

There may be included in the ethylenically unsaturated monomer diluent monomer, for instance non-ionic monomer. Such monomer may be useful to control the $pK_a$ of the acid groups, to control the hydrophilicity or hydrophobicity of the product, to provide hydrophobic regions in the polymer, or merely to act as inert diluent. Examples of non-ionic diluent monomer are, for instance, alkyl (alk) acrylates and (alk) acrylamides, especially such compounds having alkyl groups with 1 to 12 carbon atoms, hydroxy, and di-hydroxy-substituted alkyl(alk) acrylates and -(alk) acrylamides, vinyl lactams, styrene and other aromatic monomers.

In the polymer matrix, where there is ionic group present the level of ion is preferably in the range 0.1 to 10 meq $g^{-1}$, preferably at least 1.0 meq $g^{-1}$.

Where PVA macromer is copolymerised with other ethylenically unsaturated monomers, the weight ratio of PVA macromer to other monomer is preferably in the range of 50:1 to 1:5, more preferably in the range 20:1 to 1:2. In the ethylenically unsaturated monomer the ionic monomer is preferably present in an amount in the range 10 to 100 mole%, preferably at least 25 mole%.

The polymer may be formed into particles in several ways. For instance, the crosslinked polymer may be made as a bulk material, for instance in the form of a sheet or a block, and subsequently be comminuted to the desired size. Alternatively, the crosslinked polymer may be formed as such in particulate form, for instance by polymerising in droplets of monomer in a dispersed phase in a continuous immiscible carrier. Examples of suitable water-in-oil polymerisations to produce particles having the desired size, when swollen, are known. For instance US 4,224,427 describes processes for forming uniform spherical beads (microspheres) of up to 5 mm in diameter, by dispersing water-soluble monomers into a continuous solvent phase, in a presence of suspending agents. Stabilisers and surfactants may be present to provide control over the size of the dispersed phase particles. After polymerisation, the crosslinked microspheres are recovered by known means, and washed and optionally sterilised. Preferably the particles eg microspheres, are swollen in an aqueous liquid, and classified according to their size.

In the invention the pharmaceutically active agent is a non-steroidal antiinflammatory drug (NSAID). It may alternatively be defined as a COX inhibitor. The reasons for the intense pain following UFE are not currently well understood, but cells in the region of the ischemic and necrosing tissues may release a host of inflammatory markers that may give rise to prostaglandin synthesis and ensuing signalling of pain. These actives are useful as both analgesics and anti-inflammatories and thus may have a synergistic role in reducing both the cause and the effect of pain post embolisation.

Examples of specific active agents useful in the present invention are:

celecoxib (Celebrex)
rofecoxib (Vioxx)
diclofenac (Voltaren, Cataflam)
diflunisal (Dolobid)
etodolac (Lodine)
flurbiprofen (Ansaid)
ibuprofen (Motrin, Advil)
indomethacin (Indocin)
ketoprofen (Orudis, Oruvail)
ketorolac (Toradol)
nabumetone (Relafen)
naproxen (Naprosyn, Alleve)
oxaprozin (Daypro)
piroxicam (Feldene)
sulindac (Clinoril)
tolmetin (Tolectin)

The active agent is preferably a COX inhibitor. It may be selective for COX-1. The invention allows local delivery of the active to the site of embolisation, and the target fibroids. This avoids systemic delivery and the associated side effects described above with such actives, exhibited especially when the active is administered orally.

The active may be COX-2 selective. Since COX-2 inhibitors are expected to inhibit inflammation and inflammation may be induced by embolisation and hence be the cause of pain, such inhibitors are expected to be effective when delivered locally in the invention to the embolus, in the vicinity of the uterine fibroids.

Suitable COX selective inhibitors are shown in the following table:

| Log [$IC_{80}$ ratio WHMA COX-2/COX-1)] | Drugs |
|---|---|
| -2 to -1 | DFP<br>L-745337<br>Rofecoxib<br>NS398<br>Etodolac |
| -1 to 0 | Meloxicam<br>Celecoxib<br>Nimesulide<br>Diclofenac<br>Sulindac Sulphide<br>Meclofenamate<br>Tomoxiprol<br>Piroxicam<br>Diflunisal<br>Sodium Salicylate |
| 0 | Niflumic Acid<br>Zomepirac<br>Fenoprofen |
| 0 to 1 | Amypyrone<br>Ibuprofen<br>Tolmetin<br>Naproxen<br>Aspirin<br>Indomethacin<br>Ketoprofen |
| 1 to 2 | Suprofen<br>Flurbiprofen |
| 2 to 3 | Ketorolac |
| WHMA = William Harvey Human Modified Whole Blood Assay | |

The table refers to the Log [$IC_{80}$ ratio WHMA COX-2/COX-1)] for the agents which have been assayed by William Harvey Human Modified Whole Blood Assay. Those drugs with a "0" value indicate equal potency, i.e. an $IC_{80}$ ratio of 1. Values above "0" indicates the drug is more selective to COX-1 and values below "0" indicates the drug is more selective to COX-2.

DFP is Di isopropylphosphofluoridate

L-745337 is 5-methanesulphonamide-6-(2,4-difluorothiophenyl)-1-indanone.

Values from Warner T.D. et al, Proc. Natl. Acad. Sci (1999) 96, 7563.

In a further aspect of the invention there is provided a new pharmaceutical composition comprising microspheres of water-insoluble, water-swellable polymer formed by the radical polymerisation of poly(vinyl alcohol) macromer having pendant ethylenically unsaturated groups and, associated with the polymer in releasable form, a pharmaceutically active agent which is a non-steroidal anti inflammatory agent and/or which is a COX inhibitor, as defined in claim 14.

The active in this aspect is preferably a COX inhibitor, as described above in connection with the first aspect of the invention. The polymer is preferably as described above in connection with the preferred embodiment of the invention.

The pharmaceutical agent is associated with the polymer preferably so as to allow controlled release of the agent over a period. Where the agent is for reducing inflammation and pain relief this period may be up to a few days, preferably up to 72 hours when most postoperative pain is experienced. The agent may be electrostatically, or covalently bonded to the polymer or held by Van der Waal's interactions.

The pharmaceutical active may be incorporated into the polymer matrix by a variety of techniques. In one method, the active may be mixed with a precursor of the polymer, for instance a monomer or macromer mixture or a

cross-linkable polymer and cross-linker mixture, prior to polymerising or crosslinking. Alternatively, the active may be loaded into the polymer after it has been crosslinked. For instance, particulate dried polymer may be swollen in a solution of active, preferably in water or in an alcohol such as ethanol, optionally with subsequent removal of non-absorbed agent and/or evaporation of solvent. A solution of the active, in an organic solvent such as an alcohol, or, more preferably, in water, may be sprayed onto a moving bed of particles, whereby drug is absorbed into the body of the particles with simultaneous solvent removal. Most conveniently, we have found that it is possible merely to contact swollen particles suspended in a continuous liquid vehicle, such as water, with an aqueous alcoholic solution of drug, over a period, whereby drug becomes absorbed into the body of the particles. Techniques to fix the drug in the particles may increase loading levels, for instance precipitation by shifting the pH of the loading suspension to a value at which the active is in a relatively insoluble form. The swelling vehicle may subsequently be removed or, conveniently, may be retained with the particles as part of the product for subsequent use as an embolic agent or the swollen particles may be used in swollen form in the form of a slurry, i.e. without any or much liquid outside the swollen particles.

Alternatively, the suspension of particles can be removed from any remaining drug loading solution and the particles dried by any of the classical techniques employed to dry pharmaceutical -based products. This could include, but is not limited to, air drying at room or elevated temperatures or under reduced pressure or vacuum; classical freeze-drying; atmospheric pressure-freeze drying; solution enhanced dispersion of supercritical fluids (SEDS). Alternatively the drug-loaded microspheres may be dehydrated using an organic solvent to replace water in a series of steps, followed by evaporation of the more volatile organic solvent. A solvent should be selected which is a non-solvent for the drug.

In brief, a typical classical freeze drying process might proceed as follows: the sample is aliquoted into partially stoppered glass vials, which are placed on a cooled, temperature controlled shelf within the freeze dryer. The shelf temperature is reduced and the sample is frozen to a uniform, defined temperature. After complete freezing, the pressure in the dryer is lowered to a defined pressure to initiate primary drying. During the primary drying, water vapour is progressively removed from the frozen mass by sublimation whilst the shelf temperature is controlled at a constant, low temperature. Secondary drying is initiated by increasing the shelf temperature and reducing the chamber pressure further so that water absorbed to the semi-dried mass can be removed until the residual water content decreases to the desired level. The vials can be sealed, *in situ,* under a protective atmosphere if required.

Atmospheric pressure freeze drying is accomplished by rapidly circulating very dry air over a frozen product. In comparison with the classical freeze-drying process, freeze-drying without a vacuum has a number of advantages. The circulating dry gas provides improved heat and mass transfer from the frozen sample, in the same way as washing dries quicker on a windy day. Most work in this area is concerned with food production, and it has been observed that there is an increased retention of volatile aromatic compounds, the potential benefits of this to the drying of biologicals is yet to be determined. Of particular interest is the fact that by using atmospheric spray drying processes instead of a cake, a fine, free-flowing powder is obtained. Particles can be obtained which have submicron diameters, this is tenfold smaller than can be generally obtained by milling. The particulate nature, with its high surface area results in an easily rehydratable product, currently the fine control over particle size required for inhalable and transdermal applications is not possible, however there is potential in this area.

In a further aspect of the invention there is provided a new method of loading a non-steroidal anti-inflammatory agent which has an acid group as defined in claim 22 into a water-insoluble, water swellable polymer vehicle including the steps of

a) contacting water-swellable cross-linked poly(vinyl alcohol) polymer in the form of particles which are suspended in the aqueous solution which have particle sizes when equilibrated in water at 37°C in the range 100 to 1200 μm with an aqueous solution of the agent at a pH at above the pKa of the acid group,

b) adding acid to the product of step a) so as to reduce the pH of the aqueous liquid in contact with polymer to below the pKa of the acid group; and

c) recovering the polymer with loaded agent in free acid form.

Although the product of this method may be used to deliver the active by methods other than embolisation and for indications other than uterine fibroid treatment these are the preferred uses.

The new method of this aspect of the invention is of value for the COX inhibitors mentioned above whose free acid form, which is to be the form of the administered compound, is relatively water-insoluble. The compounds are naproxen, sulindac, diclofenac, indomethacin, ibuprofen, acetyl salicylate, ketorolac, ketoprofen; flurbiprofen and suprofen, preferably ibuprofen.

Preferably the pH of the aqueous solution in step a) is at least 5, and the pH of the liquid after step b) is less than 3, as the acid group is a carboxylic acid in all these compounds.

The embolic compositions of the invention may be administered in the normal manner for UFE. Thus the composition may be admixed immediately before administration by the interventional radiologist, with imaging agents such as radiopaque agents. Alternatively or additionally, the particles may be preloaded with radiopaque material in

addition to the pharmaceutical active. Thus the polymer and pharmaceutical active, provided in preformed admixture, may be mixed with a radiopaque imaging agent in a syringe, used as the reservoir for the delivery device. The composition may be administered, for instance, from a microcatheter device, into the uterine arteries. Selection of suitable particle size range, dependent upon the desired site of embolisation may be made in the normal way by the interventional radiologists.

The example is illustrated in the following examples and figures, in which

Figure 1 shows the results of the loading described in example 2 of ibuprofen from PBS;
Figure 2 shows the results of the loading of example 2 using ibuprofen in ethanol;
Figure 3 shows the release profile of ibuprofen (loaded from ethanol) into PBS from the low AMPS product in example 2;
Figure 4 shows the loading of profile of Flurbiprofen in low and high AMPS beads of example 3;
Figure 5 shows the release of Flurbiprofen from beads low and high AMPS beads of example 3;
Figure 6 shows the loading of Diclofenac in low and high AMPS beads of example 4;
Figure 7 shows the release of Diclofenac from beads of the present invention of example 4;
Figure 8 shows the ketorolac loading in low AMPS microspheres of example 5;
Figure 9 shows the release of ketorolac from low AMPS microspheres of example 5;
Figure 10 shows the loading of ibuprofen sodium salt from microspheres of example 7;
Figure 11 shows the release of ibuprofen sodium salt from microspheres of example 7;
Figure 12 shows the loading of ibuprofen free acid into microspheres of example 8;
Figure 13 shows the release of ibuprofen free acid from microspheres of example 8;
Figure 14 shows the release of ibuprofen into PBS from microspheres loaded under different conditions of example 9;
Figure 15 shows the release of ketoprofen from beads of the present invention of example 10;
Figure 16 shows the uptake of naproxen by microspheres of example 11;
Figure 17 shows the release of naproxen from microspheres of example 11; and
Figure 18 shows the release of salicylic acid from microspheres of example 12.

**Example 1: Outline Method for the Preparation of Microspheres**

*Nelfilcon B macromer synthesis:*

The first stage of microsphere synthesis involves the preparation of Nelfilcon B - a polymerisable macromer from the widely used water soluble polymer PVA. Mowiol 8-88 poly(vinyl alcohol) (PVA) powder (88% hydrolised, 12% acetate content, average molecular weight about 67,000D). (150g) (Clariant, Charlotte, NC USA) is added to a 21 glass reaction vessel. With gentle stirring, 1000ml water is added and the stirring increased to 400rpm. To ensure complete dissolution of the PVA, the temperature is raised to 99 ±9°C for 2-3 hours. On cooling to room temperature N-acryloylaminoacetaldehyde (NAAADA) (Ciba Vision, Germany) (2.49g or 0.104mmol/g of PVA) is mixed in to the PVA solution followed by the addition of concentrated hydrochloric acid (100ml) which catalyses the addition of the NAAADA to the PVA by transesterification. The reaction proceeds at room temperature for 6-7 hours then stopped by neutralisation to pH 7.4 using 2.5M sodium hydroxide solution. The resulting sodium chloride plus any unreacted NAAADA is removed by diafiltration (step 2).

*Diafiltration of macromer*:

Diafiltration (tangential flow filtration) works by continuously circulating a feed solution to be purified (in this case nelfilcon B solution) across the surface of a membrane allowing the permeation of unwanted material (NaCl, NAAADA) which goes to waste whilst having a pore size small enough to prevent the passage of the retentate which remains in circulation.

Nelfilcon B diafiltration is performed using a stainless steel Pellicon 2 Mini holder stacked with 0.1$m^2$ cellulose membranes having a pore size with a molecular weight cut off of 3000 (Millipore Corporation, Bedford, MA USA). Mowiol 8-88 has a weight average molecular weight of 67000 and therefore has limited ability to permeate through the membranes.

The flask containing the macromer is furnished with a magnetic stirrer bar and placed on a stirrer plate. The solution is fed in to the diafiltration assembly via a Masterflex LS peristaltic pump fitted with an Easy Load II pump head and using LS24 class VI tubing. The Nelfilcon is circulated over the membranes at approximately 50psi to accelerate permeation. When the solution has been concentrated to about 1000ml the volume is kept constant by the addition of water at the same rate that the filtrate is being collected to waste until 6000ml extra has been added. Once achieved, the solution is concentrated to 20-23% solids with a viscosity of 1700-3400 cP at 25°C. Nelfilcon is characterised by

GFC, NMR and viscosity.

*Microsphere Synthesis:*

The spheres are synthesised by a method of suspension polymerisation in which an aqueous phase (nelfilcon B) is added to an organic phase (butyl acetate) where the phases are immiscible. By employing rapid mixing the aqueous phase can be dispersed to form droplets, the size and stability of which can be controlled by factors such as stirring rates, viscosity, ratio of aqueous/organic phase and the use of stabilisers and surfactants which influence the interfacial energy between the phases. Two series of microspheres are manufactured, a low AMPS and a higher AMPS series, the formulation of which are shown below.

| | |
|---|---|
| A | High AMPS: |
| Aqueous: | ca 21 % w/w Nelfilcon B solution (400 ±50g approx) |
| | ca 50% w/w 2-acrylamido-2-methylpropanesulphonate Na salt (140 ±10g) |
| | Purified water (137±30g) |
| | Potassium persulphate (5.22±0.1g) |
| | Tetramethyl ethylene diamine TMEDA (6.4±0.1 ml) |
| Organic: | n-Butyl acetate (2.7 ±0.3L) |
| | 10% w/w cellulose acetate butyrate in ethyl acetate (46±0.5g) |
| | Purified water (19.0 ±0.5ml) |
| B | Low AMPS: |
| Aqueous: | ca 21% w/w Nelfilcon B solution (900 ±100g approx) |
| | ca 50% w/w 2-acryamido-2-methylpropanesulphonate Na salt (30.6 ±6g) |
| | Purified water (426±80g) |
| | Potassium persulphate (20.88±0.2g) |
| | TMEDA (25.6±0.5ml) |
| Organic: | n-Butyl acetate (2.2 ±0.3L) |
| | 10% w/w cellulose acetate butyrate (CAB) in ethyl acetate (92±1.0g) |
| | Purified water (16.7 ± 0.5ml) |

A jacketed 4000ml reaction vessel is heated using a computer controlled bath (Julabo PN 9-300-650) with feedback sensors continually monitoring the reaction temperature.

The butyl acetate is added to the reactor at 25°C followed by the CAB solution and water. The system is purged with nitrogen for 15 minutes before the PVA macromer is added. Cross linking of the dispersed PVA solution is initiated by the addition of TMEDA and raising the temperature to 55°C for three hours under nitrogen. Crosslinking occurs via a redox initiated polymerisation whereby the amino groups of the TMEDA react with the peroxide group of the potassium persulphate to generate radical species. These radicals then initiate polymerisation and crosslinking of the double bonds on the PVA and AMPS transforming the dispersed PVA-AMPS droplets into insoluble polymer microspheres. After cooling to 25°C the product is transferred to a filter reactor for purification where the butyl acetate is removed by filtration followed by:

- Wash with 2 x 300ml ethyl acetate to remove butyl acetate and CAB
- Equilibrate in ethyl acetate for 30mins then filtered
- Wash with 2 x 300 ml ethyl acetate under vacuum filtration
- Equilibrate in acetone for 30mins and filter to remove ethyl acetate, CAB and water
- Wash with 2 x 300ml acetone under vacuum filtration
- Equilibrate in acetone overnight
- Wash with 2 x 300ml acetone under vacuum.
- Vacuum dry, 2hrs, 55°C to remove residual solvents.

*Dyeing:*

This step is optional but generally unnecessary when drug is loaded with a coloured active (as this provides the colour). When hydrated the microsphere contains about 90% (w/w) water and can be difficult to visualise. To aid visualisation in a clinical setting the spheres are dyed blue using reactive blue #4 dye (RB4). RB4 is a water soluble

chlorotriazine dye which under alkaline conditions will react with the pendant hydroxyl groups on the PVA backbone generating a covalent ether linkage. The reaction is carried out at pH12 (NaOH) whereby the generated HCl will be neutralised resulting in NaCl.

Prior to dyeing, the spheres are fully re-hydrated and divided into 35g aliquots (treated individually). Dye solution is prepared by dissolving 0.8g RB4 in 2.5M NaOH solution (25ml) and water (15ml) then adding to the spheres in 2l of 80g/$l^{-1}$ saline. After mixing for 20mins the product is collected on a 32 μm sieve and rinsed to remove the bulk of the unreacted dye.

*Extraction:*

An extensive extraction process is used to remove any unbound or non specifically adsorbed RB4. The protocol followed is as shown:

- Equilibrate in 21 water for 5mins. Collect on sieve and rinse. Repeat 5 times
- Equilibrate in 21 solution of 80mM disodium hydrogen phosphate in 0.29% (w/w) saline. Heat to boiling for 30mins. Cool, collect on sieve and wash with 1l saline. Repeat twice more.
- Collect, wash on sieve the equilibrate in 21 water for 10mins.
- Collect and dehydrate in 1l acetone for 30mins.
- Combine all aliquots and equilibrate overnight in 21 acetone.

*Sieving:*

The manufactured microsphere product ranges in size from 100 to 1200 microns and must undergo fractionation through a sieving process using a range of mesh sizes to obtain the nominal distributions listed below.

1. 100-300μm
2. 300 - 500μm
3. 500 - 700μm
4. 700 - 900μm
5. 900 - 1200μm

Prior to sieving the spheres are vacuum dried to remove any solvent then equilibrated at 60°C in water to fully re-hydrate. The spheres are sieved using a 316L stainless steel vortisieve unit (MM Industries, Salem Ohio) with 15" stainless steel sieving trays with mesh sizes ranging from 32 to 1000μm. Filtered saline is recirculated through the unit to aid fractionation. Spheres collected in the 32micron sieve are discarded.

**Example 2: Uptake and Elution of Ibuprofen in Low AMPS and High AMPS Microspheres**

Two solutions were prepared, one 2.5 mg per ml of ibuprofen (in phosphate buffer solution), the second 2.5 mg per ml in ethanol. Standard curves of both solutions were measured by UV absorption at 250 nm.

**In PBS it gave Absorbance = 1.2689 x Concentration – 0.0096**

**In ethanol it gave Absorbance = 0.6875 x Concentration + 0.0329**

These standard curves were used to monitor the uptake of drug by the microspheres.

For each of the Low AMPS and High AMPS microspheres four 1 ml syringes were filled with 0.25 ml of microspheres. Two glass vials were charged with 5 ml of the 2.5 mg/ml drug in PBS and a further two vials with 5 ml of PBS to act as controls. This was repeated for the drug in ethanol and two control vials of 5 ml of ethanol, again for controls. Taking two of the Low AMPS microsphere filled syringes, the contents of one was added to the vial containing drug solution in PBS and the second syringe added to its equivalent control vial. This was repeated for two of the High AMPS microsphere filled syringes. The whole process was then repeated with the ethanol solutions.

Uptake of ibuprofen was monitored using 1 ml of solution, replaced each time to keep the concentration constant, by UV spectrometry at 250 nm. The resulting absorbencies were used to calculate the amount of drug loaded in mg per

ml of microspheres.

Absorbance (solution) - Absorbance of control = Actual Absorbance of drug loaded.

Concentration was calculated using the relevant standard curve and converted to give the concentration of drug which could be loaded into 1 ml of microspheres.

The results of the uptake from PBS over a period of one day are shown in Figure 1. The results of the uptake from ethanol are shown in Figure 2.

Release of ibuprofen from the ethanol loaded low AMPS microspheres were made in 5 ml PBS and monitored over 7 days. Concentrations were calculated using the PBS standard curve. The results are shown in Figure 3 which shows the percentage of the total released over the 7 day period.

**Example 3 : Loading and Release of Flurbiprofen from Microspheres**

A solution of 100mg/ml flurbiprofen (Sigma) in ethanol was prepared. 5 ml of the solution was added to 0.5 ml of microspheres/beads of the present invention, made as outlined in example 1. Low AMPS and high AMPS microspheres of size 500-710μm were used and drug uptake monitored by UV. The samples were agitated on a roller mixer. Aliquots of supernatant were taken at 10, 20, 30, 60 mins and then at 2hr, out to 24hr. Uptake was calculated from the flurbiprofen remaining in solution. Both types of the microspheres were loaded with similar doses of 195mg (low AMPS) and 197 (high AMPS bead) per ml of hydrated microspheres (Fig 4), and in less than 30 minutes, 99% of the drug solution is located in the microspheres. Microspheres of the present invention of each size loaded with 200mg/ml flurbiprofen were placed in 250ml water at 37°C. 30% release was achieved in first 10 minutes with a further 5% in 2 days. If microspheres were transferred to 100ml of elutant, release was slow until eventually equilibrium was reached (Fig 5).

**Example 4: Loading and Release of Diclofenac from Microspheres**

A solution of 100mg/ml diclofenac (Sigma) in ethanol was prepared. 5. ml of the solution was added to 0.5 ml of low AMPS and high AMPS microspheres of the present invention produced as outlined in example 1; both samples used microspheres having size range 500-710μm, and uptake monitored by UV. The samples were agitated on a roller mixer. Aliquots of supernatant were taken at 5,15, 30 and 240 mins and then 24hr. Uptake was calculated from the diclofenac remaining in solution. Both types of the microspheres were loaded with similar doses of 26mg (low AMPS beads) and 30mg (high AMPS beads) per ml of hydrated microspheres (Fig 6), and in less than 30 minutes, 99% of the drug solution is located in the microspheres. Microspheres of the present invention of each size loaded with 26 and 30mg/ml diclofenac were placed in 250ml water at 37°C. 18-26% release in first 5 minutes with a further 35% in 48hrs (Fig 7).

**Example 4: Loading and Release of Ketorolac from Microspheres**

Two solutions of 50mg/ml and 10mg/ml ketorolac (Sigma) in water were prepared. 5 ml of the solution was added to 0.5 ml of low AMPS microspheres, of size 500-710μm, and uptake monitored by HPLC. The samples were agitated on a roller mixer. Aliquots of supernatant were taken at 5,10,20 40 and 60 mins and then 24hr. Uptake was calculated from the ketorolac remaining in solution. The microspheres were loaded with similar approximately doses half the concentrations of the original loading solutions per ml of hydrated microspheres (Fig 8), and in less than 10 minutes, 99% of the drug solution is located in the microspheres. Microspheres of each type loaded with 13 mg and 27mg/ml ketorolac were placed in 250ml water at 37°C. From the high AMPS loaded microspheres 43% released in first 5 minutes with a 90% in 1 hrs this was followed with a slow release of a further 4% in the next 24 hrs (Fig 9). The low loaded microspheres showed a similar profile with a higher amount of ketorolac 75% released in first 5 minutes, 90% in 1 hr and a further 5% in next 24 hrs.

**Example 5: Loading and Release of Ibuprofen Free Acid from Microspheres**

A series of experiments were carried out, using a loading solution containing 250mg/ml solution of Ibuprofen free acid (Sigma) in ethanol (Romil). 2ml of this solutions was added to 1 ml of hydrated low AMPS microspheres made as described in example 1, and uptake monitored by UV of the supernatant at 263nm. The samples were agitated on a roller mixer. Samples of the supernatant were taken at 10, 20, 40, 60 mins and 24hrs. Uptake was calculated from the ibuprofen remaining in solution. The microspheres could be loaded with different doses ranging from to 142-335 mg per ml of hydrated microspheres. Elution experiments were carried out on these microspheres (table 1). Microspheres were washed to determine quick burst in various media as in table 1. Then samples were placed in 10 ml solvent and absorbance read after 10mins, a further 20 ml added and absorbance read after 10 mins, this was repeated up to 90 mls and elution was monitored up to 24hrs (table 1). Elution rate ranged between 20% - 43% with an average of 25%

in most experiments and approximately 15% was quick burst.

Table 1: Elution experiments of Ibuprofen Free Acid

| Loading solution ml | Loading mg/ml Bead | Eluted Drug (mg) | Quick Burst/Wash out Solvent | Elution Solvent Used |
|---|---|---|---|---|
| 2 | 187.08 | 47 | 100% ethanol | 50%ethanol |
| 2 | 207.7 | 53 | 50% ethanol | 50%ethanol |
| 2 | 235.53 | 60 | 100% ethanol | 0.9% Saline (pH12) |
| 2 | 177.3 | 47 | 0.9% Saline (pH12) | 0.9% Saline (pH12) |
| 2 | 185.24 | 83 | 0.9% Saline (pH12) | 0.9% Saline (pH12) |
| 2 | 142.82 | 57 | 0.9% Saline (pH12) | 0.9% Saline (pH12) |
| 3 | 323.7 | 77 | 0.9% Saline (pH12) | 0.9% Saline (pH12) |

**Example 7: Loading of Release of Ibuprofen Sodium Salt from Microspheres**

Two samples of 1 ml of hydrated Low AMPS beads (700-1100 μm, example 1) were used. For preparation of the loading solutions: a) 1 g of ibuprofen sodium salt (SIGMA) was dissolved in 4 ml of water (ROMIL) and b) 1 g of Ibuprofen sodium salt (SIGMA) was dissolved in 4 ml of ethanol (ROMIL) to give a final concentration of 250 mg/ml. Once prepared, the absorbances of the solutions were read by UV at 263 nm and dilutions were made to produce a standard curve. 2ml of the Ibuprofen solution was added to a vial containing 1 ml of beads and timing was started. The vials were placed on a roller mixer at room temperature for the entire experiment. At predetermined time points (0,10,20, 30 and 60 min) 100 μl was removed, diluted as necessary (1/200) and read at 263 nm. From the readings and the standard curve, the concentration of the solution at each time point was calculated. The amount of drug loaded onto the beads was measured by the depletion of the drug in solution when extracted with the beads. From the data the mg drug loaded per 1 ml of hydrated beads were calculated and the graph plotted. From the data shown in figure 10 it can be seen that when the ibuprofen is loaded from ethanol a maximum loading is reached in about 20 minutes before loading levels again begin to decrease. This is a consequence of a competition between drug/solvent penetration into the microspheres and a concomitant de-swelling of the beads as the ethanol dehydrates them. After 20 minutes the de-swelling becomes predominant and some of the drug solution is forced from the interstices of the bead as its structure collapses.

For elution studies, 1 ml of the 250 mg/ml loaded beads was transferred into a glass-brown container filled with 100 ml of PBS and timing was started. The containers were placed in the roller mixer at room temperature for the entire experiment. At predetermined times (15, 30, 60 and 120 minutes) 1 ml of the solution was removed, read and then placed back into the container, so the volume remained constant for the entire experiment. Samples were read at 263 nm and concentrations were calculated from the equation of the ibuprofen standard curve. From the data, the mg of drug eluted per 1 ml of hydrated beads was calculated and the graph plotted (Figure 11).

**Example 8: Loading and Elution of Ibuprofen Free Acid from Microspheres**

Five samples of 1 ml of hydrated beads Low AMPS 700 to 1100 μm were used. For each sample, 1 ml of beads in phosphate buffered saline (PBS), measured with a 10ml- glass cylinder, was transferred to a glass container and all the PBS was carefully removed with a glass Pasteur pipette. For preparing the loading solutions: 2 g of Ibuprofen free acid (SIGMA) was dissolved in 8 ml of ethanol (ROMIL) to give a final concentration of 250 mg/ml. Once prepared, the absorbances of the solution and dilutions were read by UV at 263 nm to produce a standard curve. 2ml of the ibuprofen solution was added to a vial containing 1 ml of beads (previously prepared, details above) and timing was started. This was done in duplicate; in the second experiment 1ml of ibuprofen solution was added to 1ml of ethanol (so the final concentration of the solution was 125 mg/ml).

As controls 2ml of ethanol was added to one vial and 2 ml of PBS was added to another vial, each vial containing 1ml of beads. The vials were placed on the roller mixer at room temperature for the entire experiment. At predetermined time points (0, 20, 40, 60 and 120 min) 100 μl was removed, diluted as necessary (1/200) and read at 263 nm. From the readings and the standard curve, the concentration of the solution at each time point was calculated. The amount of drug loaded onto the beads was measured by the depletion of the drug in solution. From the data the mg drug loaded per 1 ml of beads were calculated and the graph plotted (figure 12). Again, as in example 7, the contraction of the beads when exposed to ethanol causes an optimum loading to be obtained at around 20 mins before contraction causes

expulsion of the drug solution from the beads.

Loaded beads from the experiment above were used for elution experiments. 1 ml of the 250 mg/ml loaded beads was transferred into a glass-brown container filled with 20 ml of PBS and timing was started. The containers were placed in the roller mixer at room temperature for the entire experiment. At time 10 minutes, 30 ml of fresh PBS was added into the container and at time 2 h another 50 ml of PBS was added into the container to give a final volume of 100 ml. At predetermined time points (0, 5, 10, 20, 30, 45, 60, 90 min and 2, 3 and 24 hours) 1 ml of the solution was removed, read and then placed back into the container. Samples were read at 263 nm and concentrations were calculated from the equation of the ibuprofen standard curve. From the data, the mg of drug eluted per 1 ml of hydrated beads was calculated and the graph plotted (figure, 13). Controls from the experiment above were eluted in the same conditions.

**Example 9: Loading and Elution of Ibuprofen into Microspheres using pH and Solvent Triggers**

Six samples of 1 ml of beads (700-1100 μm) were used. For each sample, 1 ml of beads in phosphate buffered saline (PBS), measured with a 10ml glass cylinder, was transferred to a glass container and all the PBS was carefully removed with a glass Pasteur pipette. For preparing the loading solutions: a) 4 g of ibuprofen sodium salt (SIGMA) were dissolved in 16 ml of water (ROMIL) to give a final concentration of 250 mg/ml and b) 1 of ibuprofen free acid (SIGMA) was dissolved in 4 ml of ethanol (ROMIL) to give a final concentration of 250 mg/ml. Once prepared, the absorbances of the solution-and dilutions of the aqueous and of the alcoholic solutions were read by UV at 263 nm to produce standard curves. The aqueous loading solution of ibuprofen sodium salt was then used to load 3 samples (A, B and C) of beads. Sample A was loaded by adding 2ml of the ibuprofen salt solution to a vial containing 1 ml of hydrated beads for 20 minutes (previously prepared, details above). The vial was placed on the roller mixer at room temperature for the entire experiment Once loaded, the remaining solution was removed, measured in a graduated measurement cylinder and read at 263 nm. From the readings and the standard curve; the concentration of the solution was calculated. The amount of drug loaded onto the beads was calculated by the subtracting the amount of drug in solution from the amount in the starting loading solution. From the data the mg drug loaded per 1 ml of beads for sample A was 101 mg/ml. As a control 2ml water with no drug was "loaded" into beads.

For sample B, the loading was the same as for sample A, but, instead of the residual liquid being immediately removed, 2 ml of water at pH 1 (obtained by adding HCl to the water) was added to the vial. This was kept in the roller mixer for 20 minutes. After that, the solution was removed, and the concentration of ibuprofen remaining was determined and thus the amount loaded into the beads. The loading for sample B was found to be 129.5mg/ml loading. As control 2 ml of water at pH 1 was added to a vial containing 1 ml ff beads.

For sample C there was used 2 ml of ethanol for 20 min; after that, the solution was removed and the concentration or ibuprofen free acid remaining was determined thereby allowing calculation of the amount loaded into the bead. The amount loaded was found to be 47mg/ml bead. As control, for sample C, 2 ml of ethanol was added to a vial containing 1 ml of beads.

In sample D, 2 ml of the ethanol solution containing 250 mg/ml of ibuprofen free acid was added and kept in the roller mixer for 20 minutes. After that, the solution was removed and the concentration of ibuprofen determined. The loading of ibuprofen free acid in to the bead was found to be 110:8mg/ml.

Elution was carried out with 1 ml of the loaded beads transferred into a glass-brown container filled with 100 ml of PBS and timing was started. The containers were placed in the roller mixer at room temperature for the entire experiment. At predetermined times (15, 30, 60 and 3 and 5 hours) 1 ml of the solution was removed, read and then placed back into the container; so the volume remained constant for the entire experiment. Samples were read at 263 nm and concentrations were calculated from the equation of the ibuprofen standard curve. From the data, the amount of drug eluted per 1 ml of hydrated beads was calculated and the graph plotted (figure 14). Controls from the experiment above were eluted in the same conditions. Controls are not presented in the graphs because the concentrations eluted remained below detection limits from the entire experiment.

It can be seen that where the pH has been adjusted, release of the ibuprofen is slowed significantly. This is due to the generation of the ibuprofen free acid in-situ within the beads and hence the solubility of the drug is drastically decreased. Similarly, if the beads are exposed to ethanol after loading, the structure is collapsed due to water expulsion (as in Example 7). Upon rehydration in the buffer, the release profile of the free acid is stowed even more, suggesting that the collapsing process helps to impede drug dissolution from the polymer matrix.

**Example 10: Loading and Release of Ketoprofen from Microspheres**

A ketoprofen solution of 30mg/ml in ethanol was prepared (Sigma Aldrich). 0.5ml of 500-710μm low AMPS or high AMPS type microspheres (example 1) was added to 5ml of ketoprofen solution in duplicate (a & b), and uptake was monitored by UV over 72 hours. After an initially higher uptake which was not maintained, maximum loading occurred at 24 hours with the low AMPS microspheres showing approximately 12mg ketoprofen loaded /ml spheres and the high

AMPS microspheres showing approximately 10 mg ketoprofen loaded /ml spheres.

Release of ketoprofen from the spheres loaded for 24 hours was determined as follows: the excess loading solution was removed by glass Pasteur pipette from the loaded microspheres described above. Each sample of loaded microspheres was placed in a glass jar containing 100ml water and the jars were placed in a shaking water bath at 37°C. Release was measured by UV over 24. hours, at which point a further 100ml water was added to each jar. UV measurement was continued for 6 hours after this. Approximately 20-25% of the loaded drug was released from the microspheres, this being equivalent to approximately 2.5mg/ml of microspheres. (% calculated from the maximum loading obtained after 24 hours). This was released in the first 15 minutes of the elution. The addition of extra water after 24 hours did not bring about any further release of the drug (figure 15). There appeared to be little effect on release rate between the low and high AMPS in the microsphere formulation.

**Example 11: Loading and Release of Naproxen from Microspheres**

A naproxen solution of 30mg/ml in ethanol was prepared from naproxen obtained from Sigma Aldrich. 0.5ml of 500-710μm low AMPS or high AMPS microspheres was added to 5ml of naproxen solution in duplicate, and uptake was monitored by UV over 168 hours (7 days). The microspheres took up approximately 35-40mg naproxen /ml of spheres over 168 hours. Initial rapid uptake was followed by apparent partial release, then more gradual uptake (figure 16).

The excess loading solution was removed by glass Pasteur pipette from the loaded microspheres described in Example 8. Each sample of loaded microspheres was placed in a glass vial containing 10ml water and the vials were placed in a shaking water bath at 37°C. Release was measured by UV over 17 hours, at which point the microspheres were placed in 10ml fresh water. UV measurement were continued for 7 hours after this. Approximately 17-25% of the loaded drug was released from the microspheres, this being equivalent to approximately 6-9mg/ml of microspheres. This was released in the first 5 minutes of the elution (figure 17). The transfer of the microspheres to fresh water after 17 hours did not bring about any further release of the drug.

**Example 12: Loading and Release of Salicylic acid from Microspheres**

A salicylic acid solution of 5mg/ml in ethanol was prepared from salicylic acid obtained from Sigma Aldrich. 0.5ml of 500-710μm low AMPS or high AMPS microspheres were added to 5ml of salicylic acid solution in duplicate, and uptake was monitored by UV over 24 hours. The microspheres took up a maximum of approximately 3-4mg salicylic acid /ml of microspheres after 3-4 hours, but this had decreased to 2-3 mg/ml of microspheres after 24 hours.

The elution of the drug was assessed as follows: the excess loading solution was removed by glass Pasteur pipette from the loaded microspheres. Each sample of loaded microspheres was placed in a glass jar containing 100ml water and the vials were placed in a shaking water bath at 37°C. Release was measured by UV over 60 hours, at which point the microspheres were placed in 10ml fresh water. UV measurement were continued for 60 hours after this. The low AMPS microspheres released approximately 25% of the salicylic acid loaded, whereas the high AMPS microspheres released approximately 30% of the salicylic acid loaded. For both microsphere types the majority of the drug was released within the first 15 minutes (figure 18). The transferral of the spheres into fresh water did not bring about any further release of the drug.

**References**

Van Eijkeren MA, etal. Effects of mefenamic acid on menstrual hemostasis in essential menorrhagia. Am J Obstet Gynecol 1992; 166:1419-28.

Tannenbaum H, et al: An evidence-based approach to prescribing NSAIDs in musculoskeletal disease: a Canadian consensus. Canadian Medical Association Journal 1996; vol. 155: 77-88.

Vane JR: Mechanism of action of NSAIDs. British Journal of Rheumatology 1996; vol. 35 (suppl. 1): 1-3.

Fujiwaki R, et al. Cyclooxygenase-2 expression in endometrial cancer: correlation with microvessel count and expression of vascular endothelial growth factor and thymidine phosphorylase. Hum Pathol. 2002; 33 (2): 213-9.

Koumas L, Phipps RP. Differential COX localization and PG release in Thy-1 (+) and Thy-1 (-) human female reproductive tract fibroblasts. Am J Physiol Cell Physiol. 2002; 283(2):C599-608.

Chan WY, et al. Prostaglandins in primary dysmenorrhea. Comparison of prophylactic and nonprophylactic treatment with ibuprofen and use of oral contraceptives. Am J Med. 1981; 70(3); 535-41.

Wilson ML, Murphy PA. Herbal and dietary therapies for primary and secondary dysmenorrhoea. Cochrane Database Syst Rev. 2001; 3 CD002124.

Katz VL, et al. Complications of uterine leiomyomas in pregnancy. Obstet Gynecol. 1989; 73 (4):593-6.

Chan WY. Prostaglandins and nonsteroidal anti-inflammatory drugs in dysmenorrhea. Annu Rev Pharmacol Toxicol. 1983; 23:131-49.
Powell AM, Chan WY. Differential effects of ibuprofen and naproxen sodium on menstrual prostaglandin release and on PG production in the rat uterine homogenat. Prostaglandins Leukot Med. 1984; 13(2): 129-37.
Milsom I, etal. Intra-uterine pressure and serum ibuprofen: Observation after oral administration of 400mg ibuprofen to a patient with primary dysmenorrhoea. Eur J Clin Pharamcol. 1985; 29(4): 443-6.
Milsom I, Andresch B. Effect of ibuprofen, naproxen sodium and paracetemol on intrauterine pressure and menstrual pain in dysmenorrhoea. Br J Obstet Gynaecol. 1984; 91 (11): 1129-35.
Anderson ABM, et al. Reduction of menstrual blood-loss by prostagtandin-synthetase inhibitors. Lancet 1976; 1: 774-6.
Makarianen L etal. Primary and myoma-associated menorrhagia: role of prostaglandin and effects of ibuprofen. Br J Obstet Gynaecol. 1986; 93(9): 974-8.
Dawood MY. Dysmenorrhea and prostaglandins: pharmacological and therapeutic considerations. Drugs. 1981; 22 (1): 42-56.
Sanfilippo JS, et al. Influence of certain prostaglandin synthetase inhibitors on cytoplasmic estrogen receptors in the uterus. Am J Obstet Gynecol. 1983; 145(1): 100-4.

## Claims

**1.** Use of water-insoluble water-swellable polymer in the form of particles having particle sizes when equilibrated in water at 37°C in the range 100 to 1200 μm and, associated with polymer in a releasable form, a pharmaceutically active agent which is a non-steroidal anti-inflammatory agent selected from celecoxib, rofecoxib, diclofenac, diflunisal, etodolac, flurbiprofen, ibuprofen, indomethacin, ketoprofen, ketorolac, nabumetone, naproxen, oxaprozin, piroxicam, salicylic acid, acetyl salicylate, sulindac, tolmetin and pharmaceutically acceptable salts thereof, in the manufacture of a composition for uterine fibroid embolisation, in which the pharmaceutical active is released from the polymer at the site of embolisation.

**2.** Use according to claim 1 in which the particles are substantially spherical in shape.

**3.** Use according to claim 1, in which the active agent is a COX inhibitor selective for COX-1.

**4.** Use according to claim 1 in which the active agent is a COX inhibitor selective for COX-2.

**5.** Use according to claim 1 in which the pharmaceutically active agent is selected from ibuprofen, flurbiprofen, diclofenac, ketorolac, naproxen, ketoprofen and salicyclic acid and pharmaceutically acceptable salts thereof.

**6.** Use according to any preceding claim in which the polymer is synthetic and biostable.

**7.** Use according to any preceding claim in which the polymer is cross-linked.

**8.** Use according to claim 7 in which the polymer is covalently cross-linked.

**9.** Use according to any preceding claim in which the polymer is formed by the radical polymerisation of poly(vinyl alcohol) macromer having pendant ethylenically unsaturated groups.

**10.** Use according to claim 9 in which the pendant groups are (alk) acrylic groups.

**11.** Use according to claim 9 or 10 in which the macromer is copolymerised with ethylenically unsaturated comonomer.

**12.** Use according to claim 11 in which the comonomer is ionic comonomer.

**13.** Use according to claim 11 or claim 12 in which the comonomer is an acrylic compound.

**14.** A pharmaceutical composition comprising microspheres of water-insoluble, water-swellable polymer formed by the radical polymerisation of poly(vinyl alcohol) macromer having pendant ethylenically unsaturated groups and, associated with the polymer in releasable form, a pharmaceutically active agent which is a non-steroidal anti inflammatory agent selected from celecoxib, rofecoxib, diclofenac, diflunisal, etodolac, flurbiprofen, ibuprofen, indomethacin, ketoprofen, ketorolac, nabumetone, naproxen, oxaprozin, piroxicam, salicylic acid, acetyl salicylate, sulindac, tolmetin and salts thereof..

**15.** A composition according to claim 14 in which the active agent is a COX inhibitor selective for COX-1.

**16.** A composition according to claim 14 in which the active agent is a COX inhibitor selective for COX-2.

**17.** A composition according to claim 14 in which the pharmaceutically active agent is selected from ibuprofen, flurbiprofen, diclofenac, ketorolac, naproxen, ketoprofen and salicyclic acid and pharmaceutically acceptable salts thereof.

**18.** A composition according to any of claims 14 to 17 in which the macromer is formed by the reaction of poly(vinyl alcohol) with N-acryloylaminoacetaldehyde.

**19.** A composition according to any of claims 14 to 18 in which the macromer is copolymerised with ethylenically unsaturated comonomer.

**20.** A composition according to claim 19 in which the comonomer is ionic.

**21.** A composition according to claim 18 and claim 20 in which the comonomer is an acrylic compound.

**22.** A method of loading a non-steroidal anti-inflammatory agent, which has an acid group and which is selected from naproxen, sulindac, diclofenac, indomethacin, ibuprofen, acetyl salicylate, ketorolac, ketoprofen, flurbiprofen and suprofen, into a water-insoluble, water-swellable polymer vehicle including the steps of

a) contacting water-swellable cross-linked poly(vinyl alcohol) polymer in the form of particles which are suspended in the aqueous solution which have particle sizes when equilibrated in water at 37°C in the range 100 to 1200 μm with an aqueous solution of the agent at a pH at above the pKa of the acid group,
b) adding acid to the product of step a) so as to reduce the pH of the aqueous liquid in contact with polymer to below the pKa of the acid group; and
c) recovering the polymer with loaded agent in free acid form.

**23.** A method according to claim 22 in which the active agent is a cyclooxygenase inhibitor.

**24.** A method according to claim 23 in which the active agent is selective for COX-1.

**25.** A method according to claim 23 in which the active agent is selective for COX-2.

**26.** A method according to claim 22 in which the active agent is ibuprofen.

**27.** A method according to any of claims 22 to 26 in which the pH of the aqueous solution in step a) is at least 5, and the pH of the liquid after step b) is less than 3.

**28.** A method according to claim 22 in which the particles are substantially spherical.

**29.** A method according to any of claims 22 to 28 in which the poly(vinyl alcohol) is cross-linked by aldehyde.

**30.** A method according to any of claims 22 to 28 in which the polymer is formed by the radical polymerisation of poly (vinyl alcohol) macromer having pendant ethylenically unsaturated groups.

**31.** A method according to claim 30 in which the macromer is formed by the reaction of poly(vinyl alcohol) with N-acryloylaminoacetaldehyde.

**32.** A method according to claim 30 or 31 in which the macromer is copolymerised with ethylenically unsaturated comonomer.

**33.** A method according to claim 32 in which the comonomer is ionic.

**34.** A method according to claim 32 or 33 in which the comonomer is an acrylic compound.

**Patentansprüche**

1. Verwendung von in Wasser unlöslichem, in Wasser quellbarem Polymer in Form von Teilchen mit Teilchengrößen, wenn in Wasser bei 37°C äquilibriert, im Bereich von 100 bis 1.200 µm und, assoziiert mit dem Polymer in einer freisetzbaren Form, einem pharmazeutisch wirksamen Mittel, das ein nicht-steroidales entzündungshemmendes Mittel ausgewählt aus Celecoxib, Rofecoxib, Diclofenac, Diflunisal, Etodolac, Flurbiprofen, Ibuprofen, Indomethacin, Ketoprofen, Ketorolac, Nabumeton, Naproxen, Oxaprozin, Piroxicam, Salicylsäure, Acetylsalicylat, Sulindac, Tolmetin und pharmazeutisch akzeptablen Salzen davon ist, bei der Herstellung einer Zusammensetzung für die Uterusmyom-Embolisation, bei welcher der pharmazeutische Wirkstoff von dem Polymer an dem Ort der Embolisation freigesetzt wird.

2. Verwendung nach Anspruch 1, bei der die Teilchen im wesentlichen kugelförmig sind.

3. Verwendung nach Anspruch 1, bei der der Wirkstoff ein für COX-1 selektiver COX-Inhibitor ist.

4. Verwendung nach Anspruch 1, bei der der Wirkstoff ein für COX-2 selektiver COX-Inhibitor ist.

5. Verwendung nach Anspruch 1, bei der das pharmazeutisch wirksame Mittel aus Ibuprofen, Flurbiprofen, Diclofenac, Ketorolac, Naproxen, Ketoprofen und Salicylsäure und pharmazeutisch akzeptablen Salzen davon ausgewählt ist.

6. Verwendung nach irgendeinem vorhergehenden Anspruch, bei der das Polymer synthetisch und biostabil ist.

7. Verwendung nach irgendeinem vorhergehenden Anspruch, bei der das Polymer vernetzt ist.

8. Verwendung nach Anspruch 7, bei der das Polymer kovalent vernetzt ist.

9. Verwendung nach irgendeinem vorhergehenden Anspruch, bei der das Polymer durch Radikalpolymerisation von Poly(vinylalkohol)-Makromer mit seitenständigen ethylenisch ungesättigten Gruppen gebildet wird.

10. Verwendung nach Anspruch 9, bei der die Seitengruppen (Alk)acrylgruppen sind.

11. Verwendung nach Anspruch 9 oder 10, bei der das Makromer mit ethylenisch ungesättigtem Comonomer copolymerisiert ist.

12. Verwendung nach Anspruch 11, bei der das Comonomer ein ionisches Comonomer ist.

13. Verwendung nach Anspruch 11 oder Anspruch 12, bei der das Comonomer eine Acrylverbindung ist.

14. Pharmazeutische Zusammensetzung, umfassend Mikrokugeln aus in Wasser unlöslichem, in Wasser quellbarem Polymer, das durch Radikalpolymerisation von Poly(vinylalkohol)-Makromer mit seitenständigen ethylenisch ungesättigten Gruppen gebildet ist, und, assoziiert mit dem Polymer in freisetzbarer Form, einem pharmazeutisch wirksamen Mittel, das ein nicht-steroidales entzündungshemmendes Mittel ist, das aus Celecoxib, Rofecoxib, Diclofenac, Diflunisal, Etodolac, Flurbiprofen, Ibuprofen, Indomethacin, Ketoprofen, Ketorolac, Nabumeton, Naproxen, Oxaprozin, Piroxicam, Salicylsäure, Acetylsalicylat, Sulindac, Tolmetin und Salzen davon ausgewählt ist.

15. Zusammensetzung nach Anspruch 14, bei der der Wirkstoff ein für COX-1 selektiver COX-Inhibitor ist.

16. Zusammensetzung nach Anspruch 14, bei der der Wirkstoff ein für COX-2 selektiver COX-Inhibitor ist.

17. Zusammensetzung nach Anspruch 14, bei der das pharmazeutisch wirksame Mittel aus Ibuprofen, Flurbiprofen, Diclofenac, Ketorolac, Naproxen, Ketoprofen und Salicylsäure und pharmazeutisch akzeptablen Salzen davon ausgewählt ist.

18. Zusammensetzung nach irgendeinem der Ansprüche 14 bis 17, bei der das Makromer durch Reaktion von Poly (vinylalkohol) mit N-Acryloylaminoacetaldehyd gebildet ist.

19. Zusammensetzung nach irgendeinem der Ansprüche 14 bis 18, bei der das Makromer mit ethylenisch ungesättigtem Comonomer copolymerisiert ist.

**20.** Zusammensetzung nach Anspruch 19, bei der das Comonomer ionisch ist.

**21.** Zusammensetzung nach Anspruch 18 und Anspruch 20, bei der das Comonomer eine Acrylverbindung ist.

**22.** Verfahren zur Beladung von einem nicht-steroidalen entzündungshemmenden Mittel, das eine Säuregruppe aufweist und aus Naproxen, Sulindac, Diclofenac, Indomethacin, Ibuprofen, Acetylsalicylat, Ketorolac, Ketoprofen, Flurbiprofen und Suprofen ausgewählt ist, in ein in Wasser unlösliches, in Wasser quellbares Polymervehikel, enthaltend die Schritte

a) des Kontaktierens von in Wasser quellbarem vernetztem Poly-(vinylalkohol)-Polymer in Form von Teilchen, die in der wässrigen Lösung suspendiert sind und Teilchengrößen, wenn in Wasser bei 37°C äquilibriert, im Bereich von 100 bis 1.200 μm aufweisen, mit einer wässrigen Lösung des Mittels bei einem pH über dem pKa der Säuregruppe,
b) der Zugabe von Säure zu dem Produkt von Schritt a), um den pH der wässrigen Flüssigkeit in Kontakt mit dem Polymer auf unterhalb des pKa der Säuregruppe zu verringern; und
c) des Gewinnens des Polymers mit dem beladenen Mittel in freier Säureform.

**23.** Verfahren nach Anspruch 22, bei dem der Wirkstoff ein Cyclooxygenase-Inhibitor ist.

**24.** Verfahren nach Anspruch 23, bei dem der Wirkstoff für COX-1 selektiv ist.

**25.** Verfahren nach Anspruch 23, bei dem der Wirkstoff für COX-2 selektiv ist.

**26.** Verfahren nach Anspruch 22, bei dem der Wirkstoff Ibuprofen ist.

**27.** Verfahren nach irgendeinem der Ansprüche 22 bis 26, bei dem der pH der wässrigen Lösung in Schritt a) mindestens 5 ist und der pH der Flüssigkeit nach Schritt b) weniger als 3 ist.

**28.** Verfahren nach Anspruch 22, bei dem die Teilchen im wesentlichen kugelförmig sind.

**29.** Verfahren nach irgendeinem der Ansprüche 22 bis 28, bei dem der Poly(vinylalkohol) durch Aldehyd vernetzt ist.

**30.** Verfahren nach irgendeinem der Ansprüche 22 bis 28, bei dem das Polymer durch die Radikalpolymerisation von Poly(vinylalkohol)-Makromer mit seitenständigen ethylenisch ungesättigten Gruppen gebildet ist.

**31.** Verfahren nach Anspruch 30, bei dem das Makromer durch die Reaktion von Poly(vinylalkohol) mit N-Acryloylaminoacetaldehyd gebildet ist.

**32.** Verfahren nach Anspruch 30 oder 31, bei dem das Makromer mit ethylenisch ungesättigtem Comonomer copolymerisiert ist.

**33.** Verfahren nach Anspruch 32, bei dem das Comonomer ionisch ist.

**34.** Verfahren nach Anspruch 32 oder 33, bei dem das Comonomer eine Acrylverbindung ist.

## Revendications

**1.** Utilisation d'un polymère insoluble dans l'eau et gonflant dans l'eau sous forme de particules ayant des tailles de particules, lorsqu'elles sont équilibrées dans l'eau à 37°C, se situant dans l'intervalle de 100 à 1200 μm, et, associé avec le polymère sous une forme libérable, un agent pharmaceutiquement actif qui est un agent anti-inflammatoire non stéroïdien choisi parmi le célécoxib, le rofécoxib, le diclofénac, le diflunisal, l'étodolac, le flurbiprofène, l'ibuprofène, l'indométhacine, le kétoprofène, le kétorolac, la nabumétone, le naproxène, l'oxaprozine, le piroxicam, l'acide salicylique, l'acétylsalicylate, le sulindac, la tolmétine et leurs sels pharmaceutiquement acceptables, dans la fabrication d'une composition destinée à l'embolisation des fibroïdes utérins, dans laquelle le produit pharmaceutique actif est libéré du polymère au site de l'embolisation.

**2.** Utilisation selon la revendication 1, dans laquelle les particules ont une forme essentiellement sphérique.

**3.** Utilisation selon la revendication 1, dans laquelle l'agent actif est un inhibiteur de COX sélectif de COX-1.

**4.** Utilisation selon la revendication 1, dans laquelle l'agent actif est un inhibiteur de COX sélectif de COX-2.

**5.** Utilisation selon la revendication 1, dans laquelle l'agent pharmaceutiquement actif est choisi parmi l'ibuprofène, le flurbiprofène, le diclofénac, le kétorolac, le naproxène, le kétoprofène et l'acide salicylique et leurs sels pharmaceutiquement acceptables.

**6.** Utilisation selon l'une quelconque des revendications précédentes, dans laquelle le polymère est synthétique et biostable.

**7.** Utilisation selon l'une quelconque des revendications précédentes, dans laquelle le polymère est réticulé.

**8.** Utilisation selon la revendication 7, dans laquelle le polymère est réticulé par covalence.

**9.** Utilisation selon l'une quelconque des revendications précédentes, dans laquelle le polymère est formé par polymérisation radicalaire d'un macromère poly(alcool vinylique) ayant des groupes pendants à insaturation éthylénique.

**10.** Utilisation selon la revendication 9, dans laquelle les groupes pendant sont des groupes (alk)acryliques.

**11.** Utilisation selon la revendication 9 ou 10, dans laquelle le macromère est copolymérisé avec un comonomère à insaturation éthylénique.

**12.** Utilisation selon la revendication 11, dans laquelle le comonomère est un comonomère ionique.

**13.** Utilisation selon la revendication 11 ou la revendication 12, dans laquelle le comonomère est un composé acrylique.

**14.** Composition pharmaceutique comprenant des microsphères d'un polymère insoluble dans l'eau gonflant dans l'eau formé par polymérisation radicalaire d'un macromère poly(alcool vinylique) ayant des groupes pendants à insaturation éthylénique, et, associé avec le polymère sous une forme libérable, un agent pharmaceutiquement actif qui est un agent anti-inflammatoire non stéroïdien choisi parmi le célécoxib, le rofécoxib, le diclofénac, le diflunisal, l'étodolac, le flurbiprofène, l'ibuprofène, l'indométhacine, le kétoprofène, le kétorolac, la nabumétone, le naproxène, l'oxaprozine, le piroxicam, l'acide salicylique, l'acétylsalicylate, le sulindac, la tolmétine et leurs sels.

**15.** Composition selon la revendication 14, dans laquelle l'agent actif est un inhibiteur de COX sélectif de COX-1.

**16.** Composition selon la revendication 14, dans laquelle l'agent actif est un inhibiteur de COX sélectif de COX-2.

**17.** Composition selon la revendication 14, dans laquelle l'agent pharmaceutiquement actif est choisi parmi l'ibuprofène, le flurbiprofène, le diclofénac, le kétorolac, le naproxène, le kétoprofène et l'acide salicylique et leurs sels pharmaceutiquement acceptables.

**18.** Composition selon l'une quelconque des revendications 14 à 17, dans laquelle le macromère est formé par réaction de poly(alcool vinylique) avec du N-acryloylaminoacétaldéhyde.

**19.** Composition selon l'une quelconque des revendications 14 à 18, dans laquelle le macromère est copolymérisé avec un comonomère à insaturation éthylénique.

**20.** Composition selon la revendication 19, dans laquelle le comonomère est ionique.

**21.** Composition selon la revendication 18 et la revendication 20, dans laquelle le comonomère est un composé acrylique.

**22.** Procédé de charge d'un agent anti-inflammatoire non stéroïdien, qui a un groupe acide et qui est choisi parmi le naproxène, le sulindac, le diclofénac, l'indométhacine, l'ibuprofène, l'acétylsalicylate, le kétorolac, le kétoprofène, le flurbiprofène et le suprofène, dans un véhicule polymère insoluble dans l'eau gonflant dans l'eau, comprenant les étapes de:

a) mise en contact d'un polymère poly(alcool vinylique) réticulé gonflant dans l'eau sous forme de particules

qui sont en suspension dans une solution aqueuse et qui ont des tailles de particules, lorsqu'elles sont équilibrées dans l'eau à 37°C, se situant dans un intervalle de 100 à 1200 μm, avec une solution aqueuse de l'agent à un pH supérieur au pKa du groupe acide,
b) addition d'acide au produit de l'étape a) pour abaisser le pH du liquide aqueux en contact avec le polymère au-dessous du pKa du groupe acide; et
c) récupération du polymère avec l'agent chargé sous forme acide.

**23.** Procédé selon la revendication 22, dans lequel l'agent actif est un inhibiteur de cyclooxygénase.

**24.** Procédé selon la revendication 23, dans lequel l'agent actif est sélectif de COX-1.

**25.** Procédé selon la revendication 23, dans lequel l'agent actif est sélectif de COX-2.

**26.** Procédé selon la revendication 22, dans lequel l'agent actif est l'ibuprofène.

**27.** Procédé selon l'une quelconque des revendications 22 à 26, dans lequel le pH de la solution aqueuse dans l'étape a) est d'au moins 5, et le pH du liquide après l'étape b) est inférieur à 3.

**28.** Procédé selon la revendication 22, dans lequel les particules sont essentiellement sphériques.

**29.** Procédé selon l'une quelconque des revendications 22 à 28, dans lequel le poly(alcool vinylique) est réticulé par un aldéhyde.

**30.** Procédé selon l'une quelconque des revendications 22 à 28, dans lequel le polymère est formé par polymérisation radicalaire d'un macromère poly(alcool vinylique) ayant des groupes pendants à insaturation éthylénique.

**31.** Procédé selon la revendication 30, dans lequel le macromère est formé par réaction de poly(alcool vinylique) avec du N-acryloylaminoacétaldéhyde.

**32.** Procédé selon la revendication 30 ou 31, dans lequel le macromère est copolymérisé avec un comonomère à insaturation éthylénique.

**33.** Procédé selon la revendication 32, dans lequel le comonomère est ionique.

**34.** Procédé selon la revendication 32 ou 33, dans lequel le comonomère est un composé acrylique.

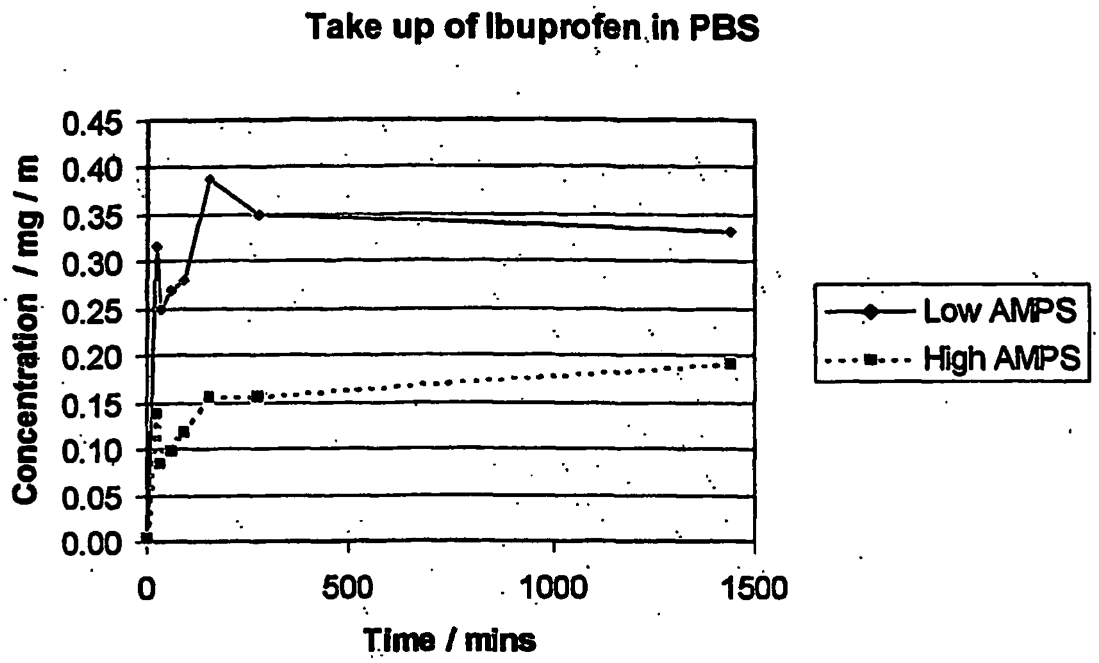


Figure 1

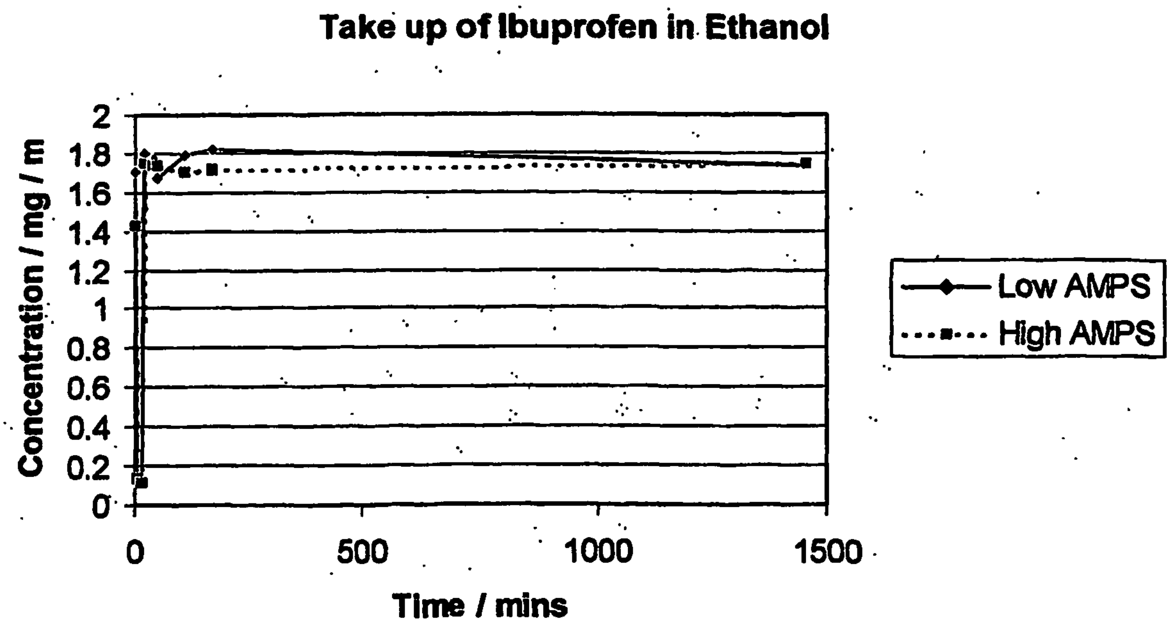


Figure 2

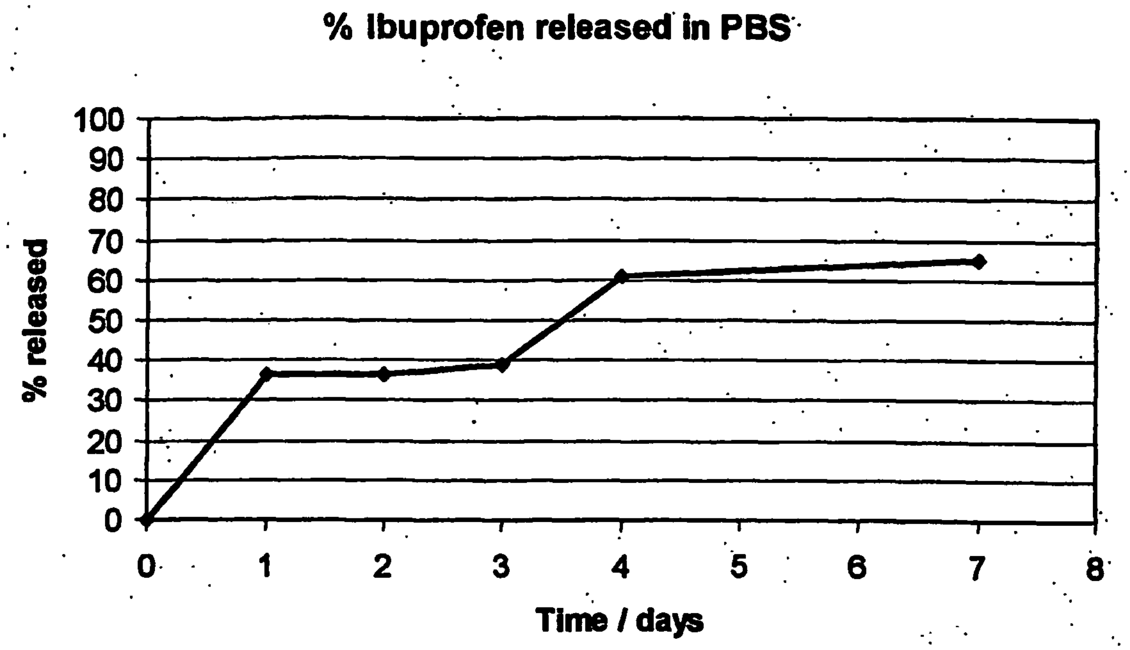
% Ibuprofen released in PBS
% released
100
90
80
70
60
50
40
30
20
10
0
0
1
2
3
4
5
6
7
8
Time / days

Figure 3

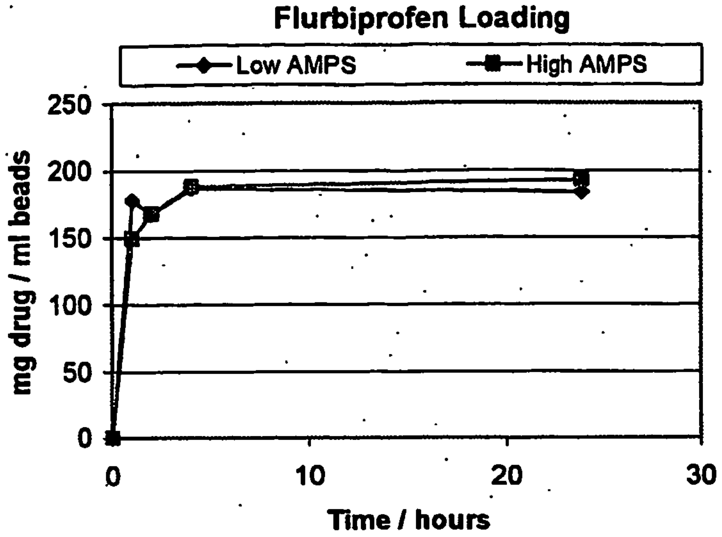


Figure 4

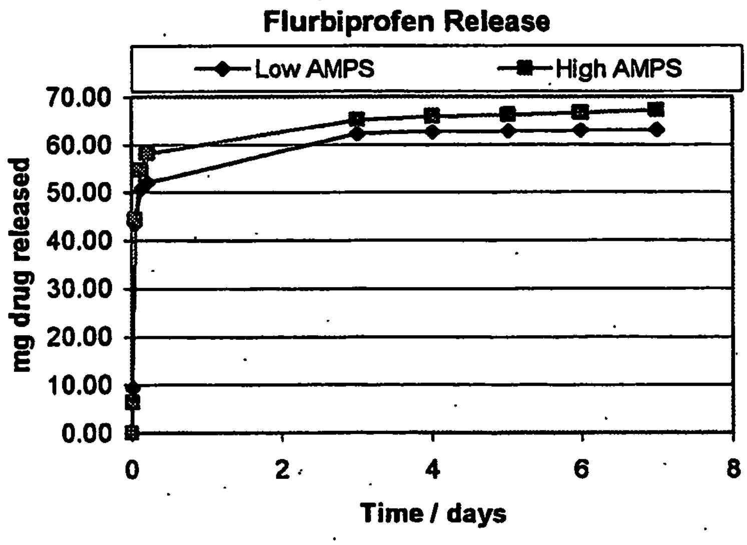


Figure 5

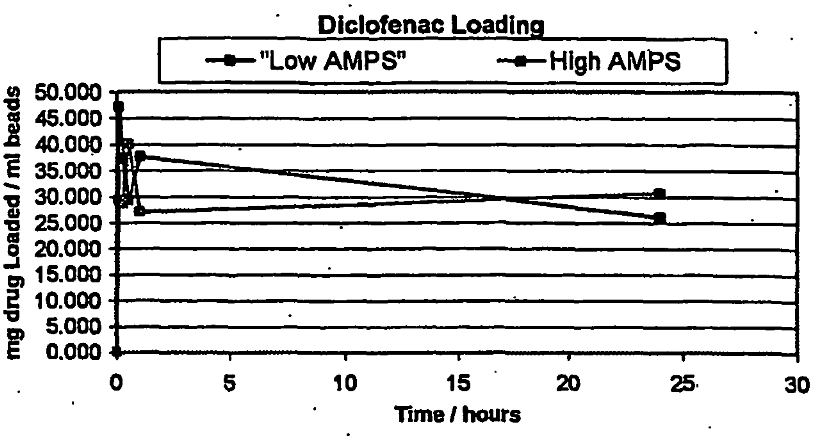


Figure 6

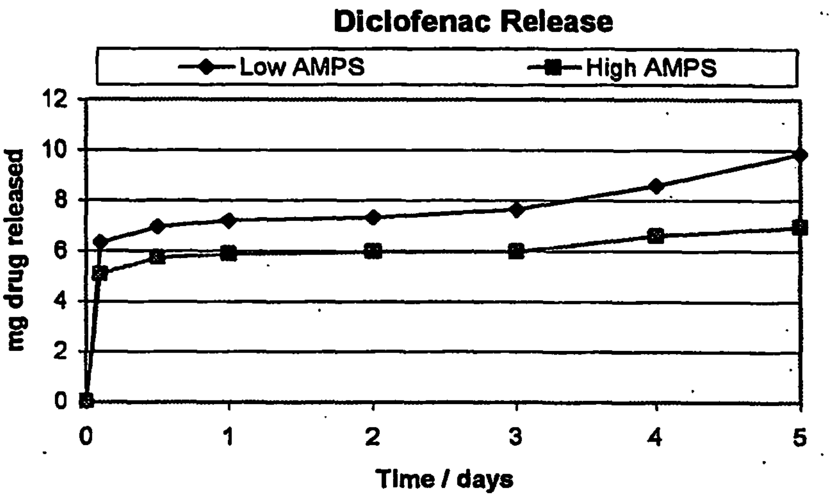


Figure 7

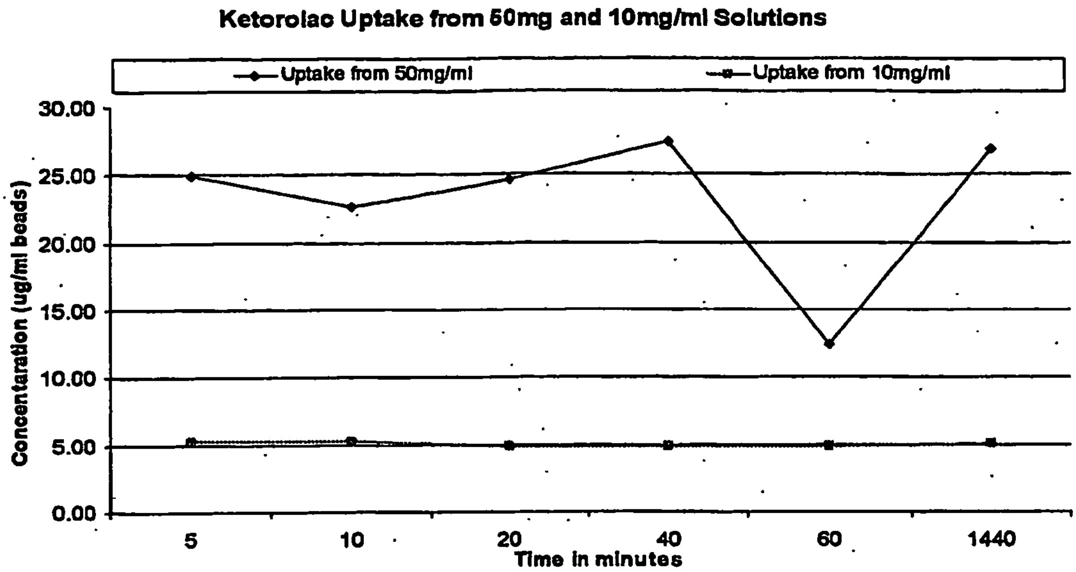


Figure 8

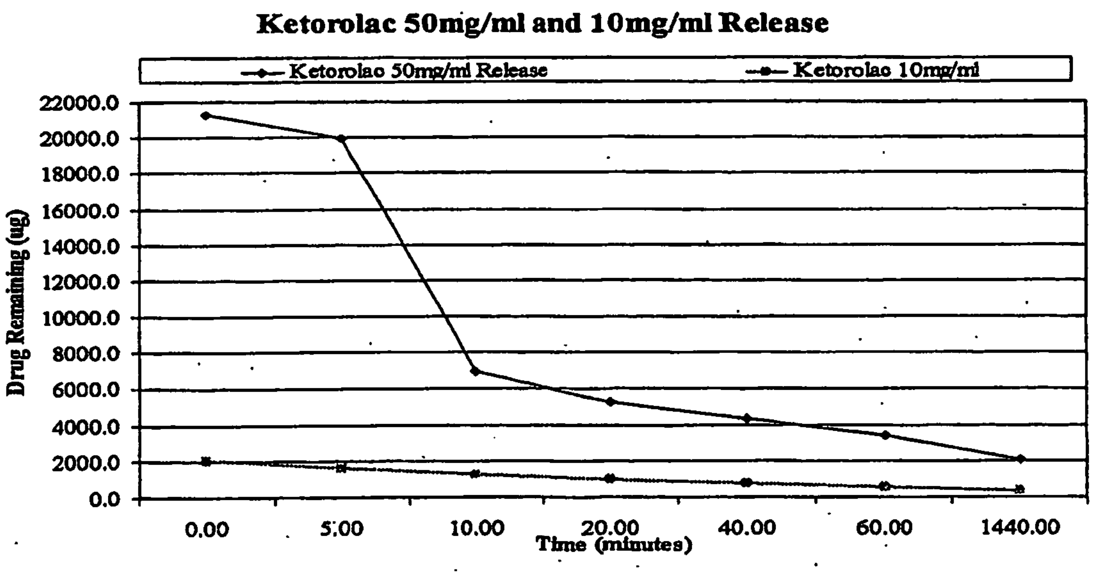


Figure 9

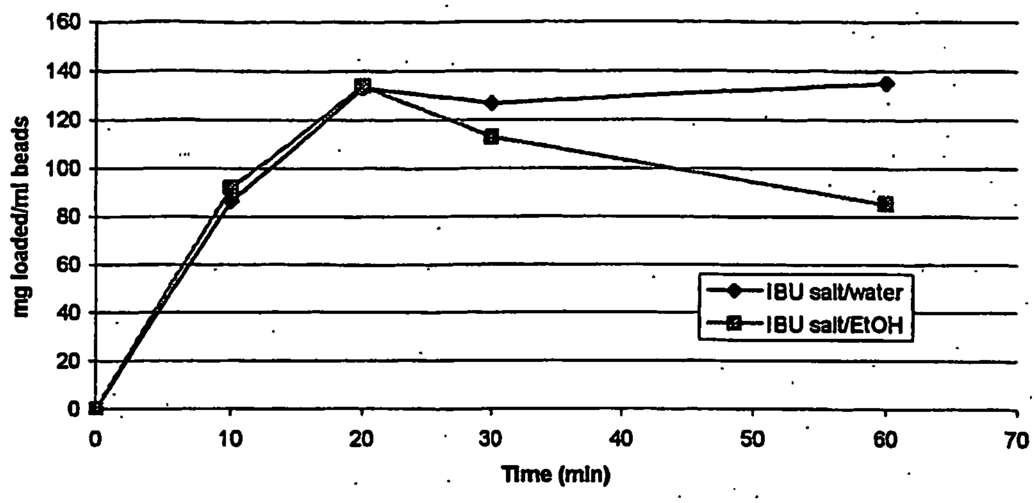


Figure 10

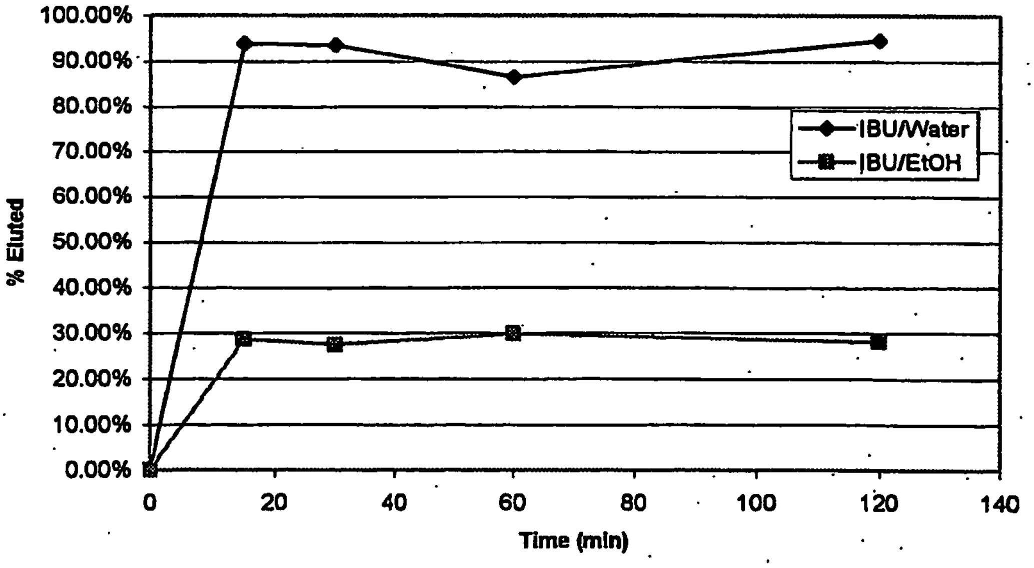


Figure 11

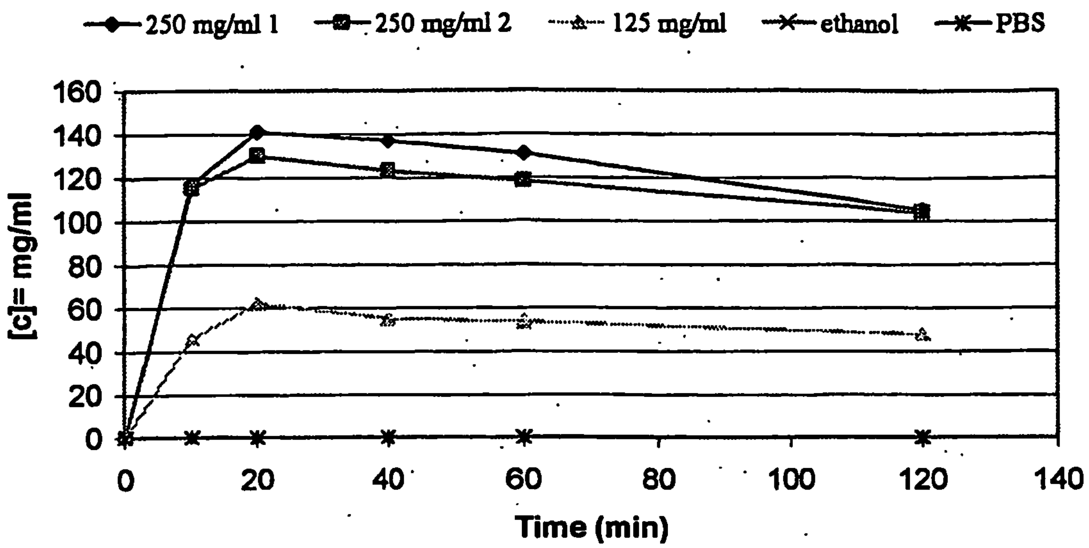


Figure 12

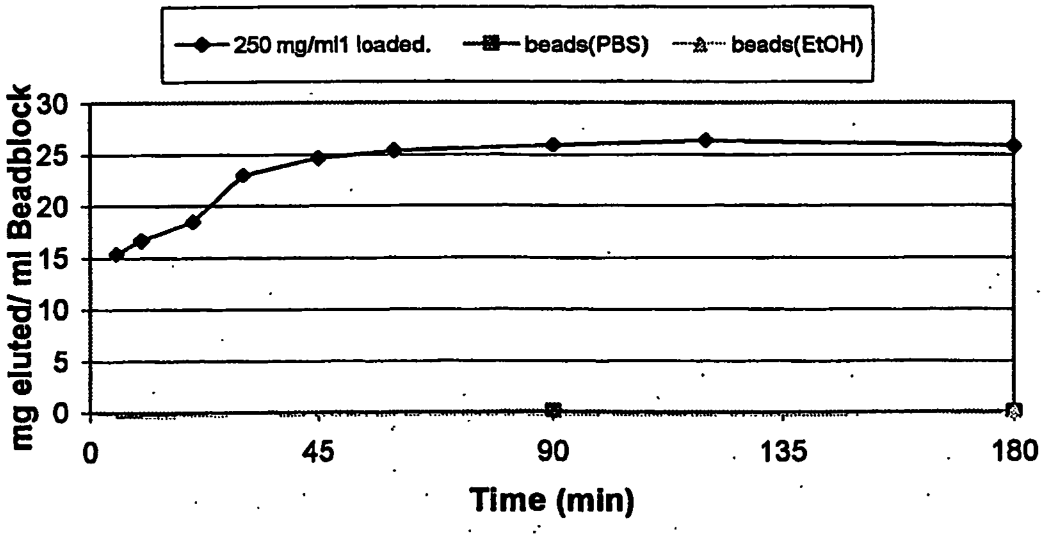


Figure 13

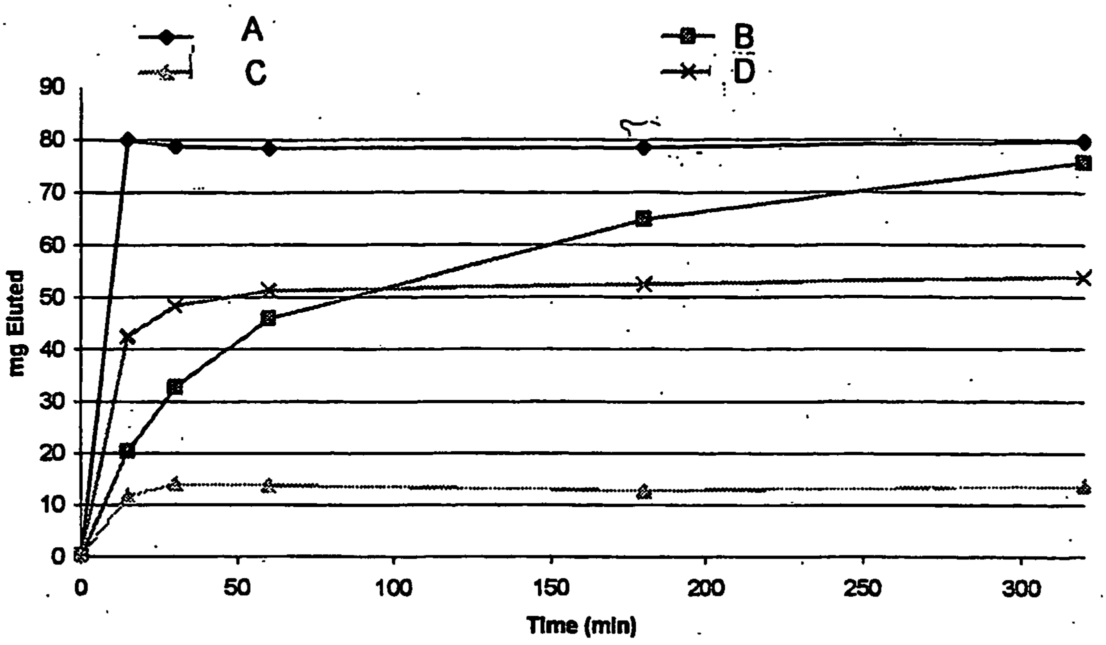


Figure 14

% Release of Ketoprofen by beads

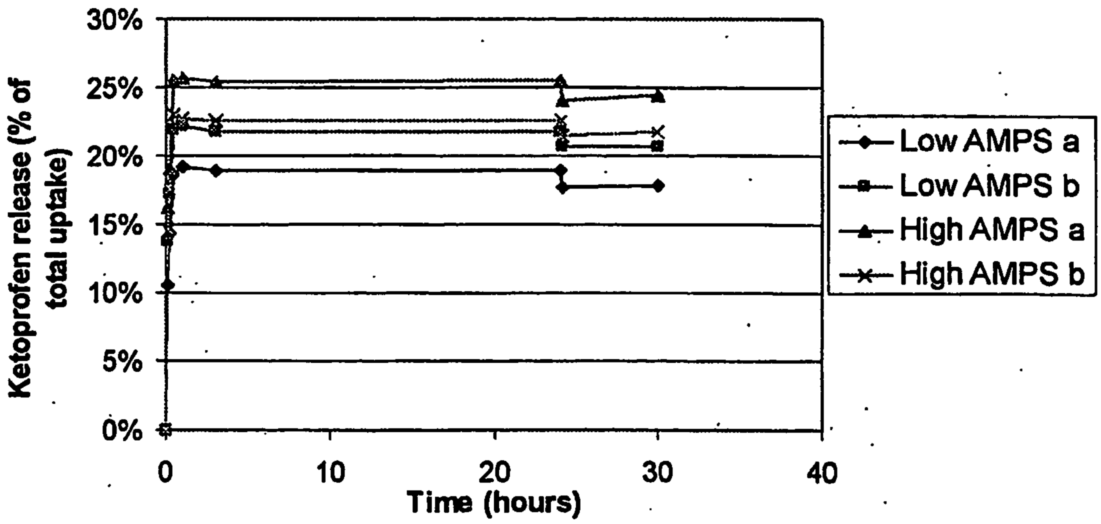


Figure 15

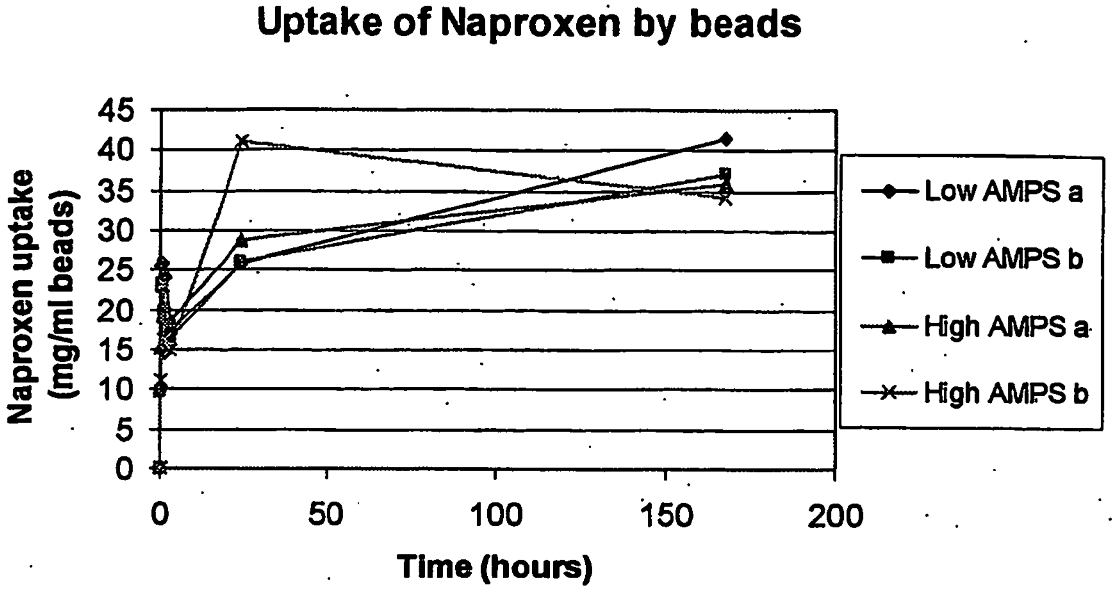


Figure 16

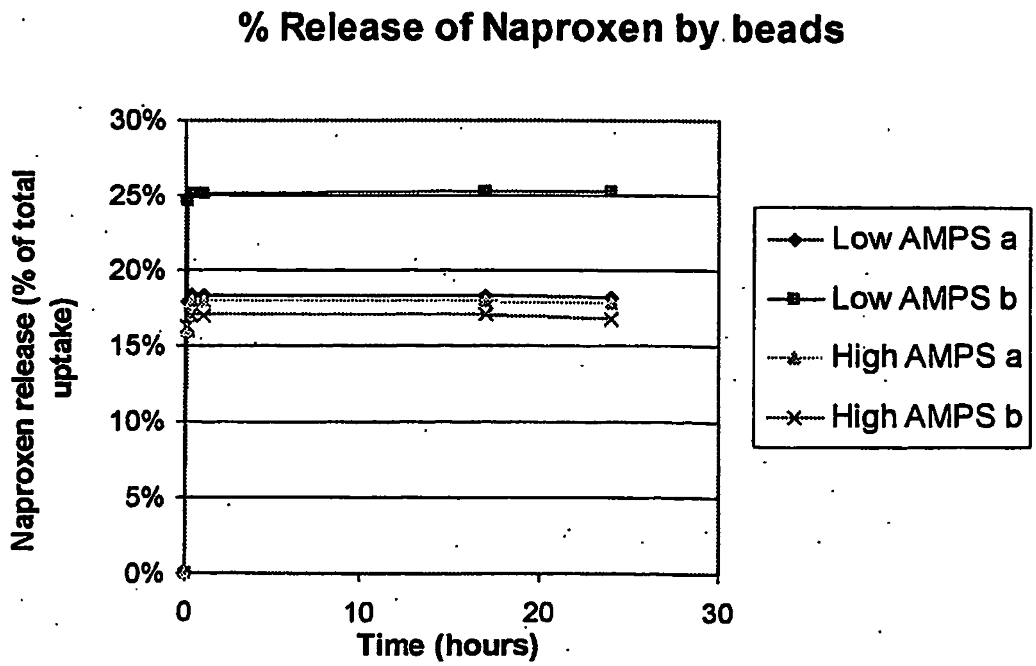


Figure 17

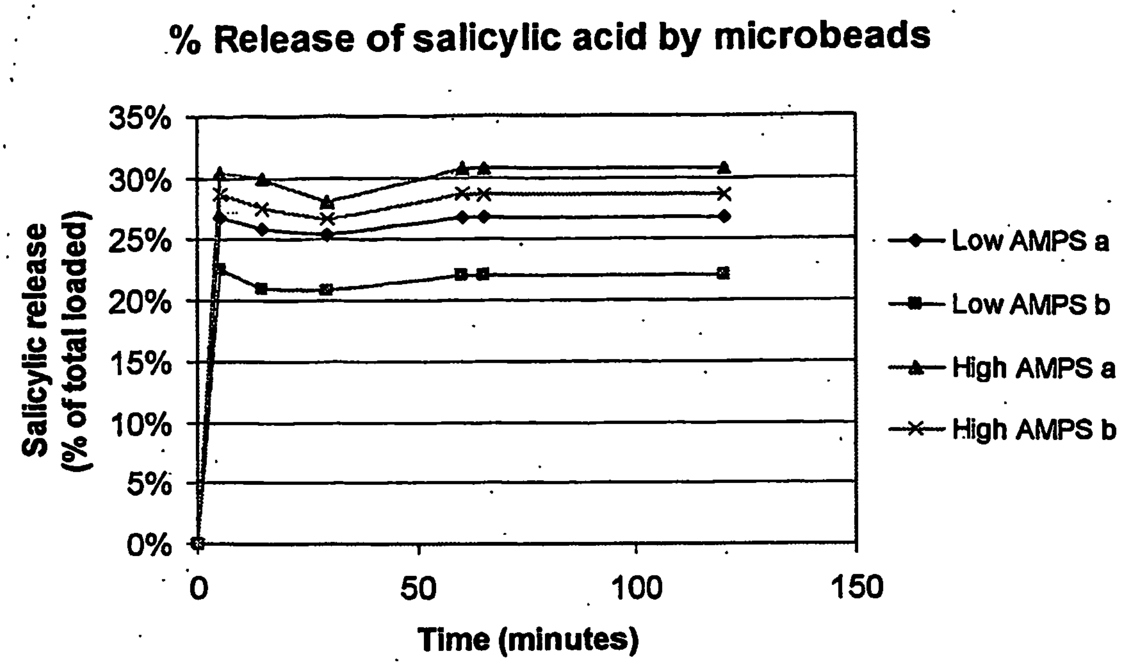
% Release of salicylic acid by microbeads
Salicylic release (% of total loaded)
35%
30%
25%
20%
15%
10%
5%
0%
0
50
100
150
Time (minutes)
Low AMPS a
Low AMPS b
High AMPS a
High AMPS b

Figure 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0023054 A
- WO 0168720 A
- US 4978713 A
- US 5508317 A
- US 5583163 A
- WO 0029481 A
- WO 9207885 A
- WO 9416748 A
- WO 9416749 A
- WO 9520407 A
- US 4224427 A


### Non-patent literature cited in the description


- **VEDANTHAM et al.** *Appl Radiol,* 17 September 2002, vol. 31 (10
- **KEYOUNG JA ; LEVY EB ; ROTH AR et al.** Intraarterial lidocaine for pain control after uterine artery embolization for leiomyomata. *J vasc Intervent Radiol,* 2001, vol. 12, 1065-1069
- **GOODWIN SC ; MCLUCAS B ; LEE M et al.** Uterine artery embolization for the treatment of uterine leiomyomata midterm results. *J Vasc Intervent Radiol,* 1999, vol. 10, 1159-1165
- **SISKIN GP ; STAINKEN BF ; DOWLING K et al.** Outpatient uterine artery embolization for symptomatic uterine fibroids: Experience in 49 patients. *J Vasc Intervent Radiol,* 2000, vol. 11, 305-311
- **BURBAK F et al.** *J Am Soc Gyn Laparoscopists,* 2000, vol. 7 (4), 1-49
- **SISKIN et al.** *Techniques in Vascular and Interventional Radiology,* 2002, vol. 5 (1), 35-43
- **WARNER T.D et al.** *Proc. Natl. Acad. Sci,* 1999, vol. 96, 7563
- **VAN EIJKEREN MA et al.** Effects of mefenamic acid on menstrual hemostasis in essential menorrhagia. *Am J Obstet Gynecol,* 1992, vol. 166, 1419-28
- **TANNENBAUM H et al.** An evidence-based approach to prescribing NSAIDs in musculoskeletal disease: a Canadian consensus. *Canadian Medical Association Journal,* 1996, vol. 155, 77-88
- **VANE JR.** Mechanism of action of NSAIDs. *British Journal of Rheumatology,* 1996, vol. 35 (1), 1-3
- **FUJIWAKI R et al.** Cyclooxygenase-2 expression in endometrial cancer: correlation with microvessel count and expression of vascular endothelial growth factor and thymidine phosphorylase. *Hum Pathol,* 2002, vol. 33 (2), 213-9
- **KOUMAS L ; PHIPPS RP.** Differential COX localization and PG release in Thy-1 (+) and Thy-1 (-) human female reproductive tract fibroblasts. *Am J Physiol Cell Physiol.,* 2002, vol. 283 (2), C599-608
- **CHAN WY et al.** Prostaglandins in primary dysmenorrhea. Comparison of prophylactic and nonprophylactic treatment with ibuprofen and use of oral contraceptives. *Am J Med,* 1981, vol. 70 (3), 535-41
- **WILSON ML ; MURPHY PA.** Herbal and dietary therapies for primary and secondary dysmenorrhoea. *Cochrane Database Syst Rev,* 2001, vol. 3
- **KATZ VL et al.** Complications of uterine leiomyomas in pregnancy. *Obstet Gynecol,* 1989, vol. 73 (4), 593-6
- **CHAN WY.** Prostaglandins and nonsteroidal anti-inflammatory drugs in dysmenorrhea. *Annu Rev Pharmacol Toxicol,* 1983, vol. 23, 131-49
- **POWELL AM ; CHAN WY.** Differential effects of ibuprofen and naproxen sodium on menstrual prostaglandin release and on PG production in the rat uterine homogenat. *Prostaglandins Leukot Med,* 1984, vol. 13 (2), 129-37
- **MILSOM I et al.** Intra-uterine pressure and serum ibuprofen: Observation after oral administration of 400mg ibuprofen to a patient with primary dysmenorrhoea. *Eur J Clin Pharamcol,* 1985, vol. 29 (4), 443-6
- **MILSOM I ; ANDRESCH B.** Effect of ibuprofen, naproxen sodium and paracetemol on intrauterine pressure and menstrual pain in dysmenorrhoea. *Br J Obstet Gynaecol,* 1984, vol. 91 (11), 1129-35
- **ANDERSON ABM et al.** Reduction of menstrual blood-loss by prostagtandin-synthetase inhibitors. *Lancet,* 1976, vol. 1, 774-6
- **MAKARIANEN L et al.** Primary and myoma-associated menorrhagia: role of prostaglandin and effects of ibuprofen. *Br J Obstet Gynaecol,* 1986, vol. 93 (9), 974-8
- **DAWOOD MY.** Dysmenorrhea and prostaglandins: pharmacological and therapeutic considerations. *Drugs,* 1981, vol. 22 (1), 42-56

- **SANFILIPPO JS et al.** Influence of certain prostaglandin synthetase inhibitors on cytoplasmic estrogen receptors in the uterus. *Am J Obstet Gynecol,* 1983, vol. 145 (1), 100-4